# EUROPÄISCHE PATENTSCHRIFT

(11) **EP 1 919 509 B1**
(45) Veröffentlichungstag und Bekanntmachung des Hinweises auf die Patenterteilung: **14.05.2014**
(21) Anmeldenummer: 06791773.2
(22) Anmeldetag: 31.08.2006
(51) Int. Cl.: A61K 47/48, C07C 323/52, C07C 323/60, A61K 31/40, C07C 237/08

(54) **HOCH VERZWEIGTE REAGENZIEN ZUR MODIFIKATION VON BIOPHARMAZEUTIKA, DEREN HERSTELLUNG UND ANWENDUNG**
HIGHLY CROSS-LINKED REAGENTS FOR MODIFYING BIOPHARMACEUTICALS, PRODUCTION AND USE THEREOF
REACTIFS HAUTEMENT RAMIFIES DESTINES A LA MODIFICATION DE PRODUITS BIOPHARMACEUTIQUES, LEUR PRODUCTION ET LEUR UTILISATION

(30) Priorität: 01.09.2005 DE 102005041570
(43) Veröffentlichungstag der Anmeldung: 14.05.2008
(73) Patentinhaber: celares GmbH, 13125 Berlin (DE)
(72) Erfinder: KRÄHMER, Ralf, 13189 Berlin (DE); LEENDERS, Frank, 13187 Berlin (DE)
(74) Vertreter: Sommer, Andrea
(86) Internationale Anmeldenummer: PCT/EP2006/008538
(87) Internationale Veröffentlichungsnummer: WO 2007/025763

(56) Entgegenhaltungen:
- WO-A-98/18494
- WO-A2-02/43772
- WO-A2-2004/083258
- WO-A2-2004/108634
- US-A1- 2003 161 791

## Beschreibung

Die vorliegende Erfindung betrifft Verbindungen, welche zur Kopplung an Pharmazeutika, insbesondere Biopharmazeutika geeignet sind, sowie Konjugate aus den Verbindungen mit Biomolekülen oder pharmazeutischen Wirkstoffen. Die erfindungsgemäßen Verbindungen sind hoch verzweigt und können auf einfache Weise durch Verwendung eines zentralen Verzweigungsbausteins gebildet werden. Ein weiterer Gegenstand der Erfindung ist die Verwendung der Konjugate als verbesserte Formulierung von Pharmazeutika.

Die Entwicklung von Biopharmazeutika als Arzneistoffe oder für potentielle Arzneistoffe sowie biotechnologischer Produkte für die Anwendung im Bereich Proteomics oder technischen Bereich machte in den vergangenen Jahrzehnten sprunghafte Fortschritte, die durch mehrere Faktoren entscheidend beeinflusst wurden:
a) verbesserte Isolierungs- und Reinigungstechniken,
b) die revolutionären Entwicklungen in der Gentechnologie und damit verbunden die Möglichkeit zur Herstellung rekombinanter Proteine,
c) das bessere Verständnis der Biochemie sowie der Wirkmechanismen von Biophamazeutika und
d) die Erschließung neuer Anwendungsgebiete und Methoden für biotechnologische Produkte.

Die Effektivität und die Dauer der Wirkung eines Wirkstoffs werden durch sein pharmakologisches Profil bestimmt. Besonders bei Biopharmazeutika wird sehr oft *in vivo* ein schneller Wirkungsverlust beobachtet, was allgemein als "clearance" bezeichnet wird. Die clearance erfolgt durch Prozesse wie Verstoffwechselung bzw. Metabolisierung, renale Ausscheidung und durch die Reaktion des Immunsystems auf die körperfremde Verbindung. Gerade proteinogene Wirkstoffe, eine wichtige Gruppe von Biopharmazeutika, rufen bei deren therapeutischem Einsatz eine starke Immunantwort hervor, die bis zum allergischen Schock führen kann. Solche nachteiligen Effekte verhindern in vielen Fällen den kommerziellen bzw. therapeutischen Einsatz dieser sonst sehr vorteilhaften Wirkstoffklasse.

Wissenschaftler beschäftigen sich seit vielen Jahren damit, Strategien zu entwickeln, den therapeutischen Einsatz von Biopharmazeutika dennoch zu ermöglichen. Eine der ersten Methoden war die Änderung der Oberflächenladung durch Umsetzung von Proteinen mit Bernsteinsäureanhydrid. Diese Modifikation wird als Succinylierung bezeichnet (Habeeb, A.F.S.A. Arch. Biochem. Biophys. 1968, 121, 652). Eine kovalente Bindung einer biologisch aktiven Verbindung an verschiedenste Polymere stellt eine der erfolgreichsten Strategien der letzten Jahre dar und wurde zu einer der wichtigsten Methoden zur Verbesserung der pharmakologischen und toxikologischen Eigenschaften von biopharmazeutischen Wirkstoffen. Eines der am häufigsten verwendeten Polymere ist hierbei das Polyalkylenoxid Polyethylen-glykol, kurz PEG genannt.

WO 2004/108634 beschreibt Verbindungen, welche zur Kopplung an Pharmazeutika, insbesondere Biopharmazeutika geeignet sind, sowie Konjungate aus den Verbindungen mit Biomolekülen oder pharmazeutischen Wirkstoffen, wobei die Verbindungen durch Mehrkomponentenreaktionen gebildet werden.

Abuchowski, einer der Pioniere auf dem Gebiet der polymervermittelten Darreichung von Biopharmazeutika, konnte zeigen, dass eine kovalente Kopplung von Polyethylenglykolketten an ein Polypeptidmolekül einen positiven pharmakologischen Effekt bei diesem Wirkstoff erzeugt. Ein solches Konjugat zeichnet sich durch eine verminderte Immunogenität und eine verlängerte Halbwertszeit im Blut aus (US-Patent Nr. 4 179 337, Davis et al.; Abuchowski & Davis "Enzymes as Drugs", Holcenberg & Roberts, Hrsg. John Wiley & Sons, N.Y. 1981, 367-383). Ferner hat die Modifikation biotechnologischer Produkte, wie z.B. Enzyme, häufig Einfluss auf weitere biochemische Parameter, z.B. auf deren pH- und Thermostabilität. Eine Modifikation kann deshalb z.B. für technische Enzyme zum Einsatz in Waschmitteln aufgrund einer erhöhten Thermostabilität oder für Biopharmazeutika im Hinblick auf eine orale Verabreichung aufgrund einer erhöhten pH-Stabilität vorteilhaft sein.

Die oben beschriebenen Arbeiten beschleunigten massiv die Forschung auf dem Gebiet der Konjugation von Wirkstoffen mit dem Polymer Polyethylenglykol. Die Modifikation mit Polyethylenglykol bietet auch bei herkömmlichen kleinen Wirkstoffen einige Vorteile. Die kovalente Bindung eines kleinen Wirkstoffs an das hydrophile Molekül Polyethylenglykol erhöht die Löslichkeit des Konjugates und kann außerdem die Toxizität verringern (Kratz, F. et al. Bioorganic & Medicinal Chemistry 1999, 7, 2517-2524). Die wichtigsten Übersichtsartikel zur Konjugation mit Polyethylenglykol sind im Folgenden benannt: Greenwald, R.B., J. of Controlled Release 2001, 74, 159-171; Zalipsky, S. Advanced Drug Delivery Reviews, 1995, 16, 157-182; Zalipsky, S. Bioconjugate Chem. 1995, 6, 150-165; N. K. Jain, N.K.; et al. Pharmazie 2002, 57, 5 - 29.

EP 1 400 550 beschreibt verzweigte Polyalkylenglykole, bei welchen die Polyethylengruppierung direkt über eine Carbamatgruppe an die Verzweigungsstruktur, bei welcher es sich um TRIS handeln kann, gebunden ist. Es wird die Verwendung der verzweigten Polyalkylenglykole als Modifikatoren für physiologisch aktive Polypeptide beschrieben.

Die chemische Reaktion zur Kopplung eines Polyethylenglykolmoleküls an ein Biopharmazeutikum erfordert die Aktivierung einer der beiden Komponenten, die zur Reaktion gebracht werden. Im Regelfall wird hierzu das PEG-Molekül mit einem Verbindungsmolekül, dem so genannten aktivierten Linker versehen. Zur Aktivierung steht die gesamte Breite der lange etablierten Peptidchemie zur Verfügung. Zur Modifikation von Aminofunktionalitäten, meist von Lysinresten als Baustein einer biologisch aktiven Verbindung, wird der Linker häufig in Form eines N-Hydroxysuccinimid Aktivesters aktiviert. Harris, J.M. et al. (US-Patent Nr. 5,672,662) entwickelte diese Methode für Propion- und Buttersäurelinker, während bei Zalipsky, S. et al. (US-Patent Nr. 5,122,614) ein aktivierter Kohlensäureester zum Einsatz kommt. Die Reaktion eines Lysinrestes mit einem solchen aktivierten Linker führt zur Bildung einer Amid- bzw. Urethanbindung. Die Verknüpfung eines PEGs mit einem Biopharmazeutikum, PEGylierung genannt, führt in manchen Fällen zum Verlust der biologischen Aktivität. Ein Grund dafür kann der Verlust der positiven Ladung durch die Bildung einer Amidbindung am Lysinrest sein.

Die reduktive Aminierung unter Verwendung von PEG-Aldehyden stellt eine gute Alternative zu der Verwendung von Aktivestern dar (Harris, J.M. US-Patent Nr. 5,252,714), weil diese Kopplungsmethode zur Bildung eines sekundären Amins unter Erhalt der positiven Ladung führt. Weitere Kopplungsmöglichkeiten bestehen in der Verwendung der Maleimidmethode (Cysteinreste) und der direkten Verbindung ohne eine Linkergruppe beim Einsatz von Tresyl- oder Halogenverbindungen.

Bei den am häufigsten verwendeten PEGs handelt es sich um lineare Monomethoxypolyethylenglykol-Ketten (m-PEGs). Diese sind kommerziell mit Molekulargewichten von 1000 Da bis 30.000 Da erhältlich. Für verzweigte m-PEG-Reagenzien gibt es bisher erst wenige kommerzielle Beispiele. Dabei wäre es aus verschiedenen Gründen technisch und wirtschaftlich sinnvoll, mehrfach verzweigte PEG-Reagenzien einzusetzen:
a) Herstellungsbedingt nimmt die Qualität von m-PEGs mit zunehmendem Molekulargewicht ab. Insbesondere ab 30.000 Da ist die Qualität nicht mehr hinreichend für den Einsatz in der pharmazeutischen Industrie. Da für viele Anwendungen jedoch Molekulargewichte deutlich > 30.000 Da benötigt werden, müssen mehrere lineare m-PEGs zu einem verzweigten Molekül kombiniert werden. Dies wird heute z.B. durch ein mit zwei m-PEG-Ketten versehnes, aktiviertes Lysin realisiert.
b) Die heute kommerziell verfügbaren m-PEGs können wirtschaftlich nur bis zu einem Molekulargewicht von 1000 Da monodispers, d.h. mit einer einheitlichen, jederzeit reproduzierbaren Qualität, hergestellt werden.
c) Lineare Ketten sind konformativ nicht eingeschränkt und können sich je nach Umgebung frei drehen. Folglich ist die von den Ketten abgeschirmte Oberfläche des Biopharmazeutikums relativ gering. Um die Oberflächenabschirmung zu verbessern, werden verzweigte Modifizierungsreagenzien entwickelt, die mehrere PEG-Ketten in einem Molekül enthalten.

Bisher werden für die Herstellung von verzweigten Polymer-Reagenzien meist Aminosäuren als Verzweigungsgruppe verwendet. Glutaminsäure oder Lysin bieten die Möglichkeit, mPEGs an zwei stellen einzuführen und die Verzweigungsgruppe anschließend an einer Position für die Kopplung an den Wirkstoff zu aktivieren. Dieses Verfahren erlaubt jedoch nur die Herstellung einfach verzweigter, so genannter "Y-shaped" Polymer-Reagenzien (Harris et. al. US Patent Anmeldung US 2003/0114647 und US Patent 5,932,462; Greenwald et. al. US Patent 6,113,906; Martinez et. al. US Patent 5,643,575). Diese Y-shaped Polymer-Reagenzien besitzen jedoch aufgrund der auch hier vorhanden freien Drehbarkeit der Bindungen nur einen mäßig verbesserten Abschirmeffekt gegenüber linearen Polymer-Reagenzien (Veronese, F.M. Bioconjugate Chem. 1995, 6, 62-69).

Eine Alternative sind verzweigte Polymer-Reagenzien, die durch direkte Kopplung von zwei m-PEGs an eine nicht planare, zyklische Komponente hergestellt werden (Yamasaki, M. et. al. EP1,270,642 A1). Durch die zyklische Struktur der Verzweigungskomponente werden eine bessere Aufspreizung der beiden m-PEG-Ketten und damit ein verbesserter Oberflächenschutz erreicht. Defrees F. beschreibt eine erweiterte Methode, indem die Verzweigung durch Verwendung eines Dipeptids und im weiteren durch Kopplung an ein Kohlenwasserstoff wie Galaktose noch weiter erhöht werden kann (Defrees F. PCT Anmeldung WO2004/083258).

Krähmer et. al. haben erstmals eine Synthese beschrieben, die in einem Schritt die Herstellung mehrfach verzweigter Polymer-Reagenzien mittels einer Mehrkomponenten-Reaktion, z.B. der Ugi-Reaktion, erlaubt (PCT/EP2004/006135). Die Verzweigung wird erst während der Synthese aufgebaut und ist somit nicht durch die Verwendung von kommerziell verfügbaren natürlichen Aminosäuren eingeschränkt. Die Autoren konnten nachweisen, dass eine Verzweigung wesentliche Vorteile hinsichtlich der angestrebten Funktion zur Wirkstoffformulierung als auch der Qualität der Reagenzien ermöglicht. Allerdings können mittels dieses Verfahrens derzeit nur Polymer-Reagenzien mit 4 Ketten wirtschaftlich hergestellt werden, weil die eingesetzte Mehrkomponenten-Reaktion nur begrenzt oft wiederholt werden kann.

Bisher ungelöst ist die wirtschaftliche Herstellung hoch verzweigter Polymer-Reagenzien, die insbesondere mehr als vier mPEG-Ketten enthalten können und nicht auf der Verwendung von natürlichen L-Aminosäuren basieren.

Ein weiterer wesentlicher Aspekt für die pharmazeutische Anwendung ist die Qualität von Polymer-Reagenzien für die PEGylierung. Polymere liegen herstellungsbedingt als heterogenes Gemisch vor. Dies wird als Polydispersität bezeichnet und beschreibt die Mischung verschiedener Kettenlängen und Molekulargewichte in einer Polymer-Population. Bei der Herstellung pharmazeutischer Produkte ist es natürlich wünschenswert, Polymere mit möglichst geringer Heterogenität zu haben, um die Gleichförmigkeit und Reproduzierbarkeit von Produktions-Batches zu gewährleisten. Es sind bisher jedoch nur wenige Methoden beschrieben, wie man zu entsprechenden monodispersen Polymer-Reagenzien kommt, die für die pharmazeutischen Anwendung geeignet sind. Wirtschaftlich sind monodisperse mPEGs mit den verfügbaren Methoden nur bis zu einem Molekulargewicht von 1000 Da herstellbar.

Aufgrund der beschriebenen Defizite beim Stand der Technik gibt es einen großen Bedarf an hoch verzweigten Modifizierungsreagenzien, mit denen Molekulargewichte von mehr als 40kDa erreicht werden können, die eine hohe und jederzeit reproduzierbare Qualität aufweisen, eine verbesserte Schutzfunktion für den modifizierten Wirkstoff zeigen und wirtschaftlich im großen Maßstab herstellbar sind.

Die Aufgabe der Erfindung bestand deshalb darin, Verbindungen bereitzustellen, welche an Biopharmazeutika gebunden werden können und mit denen die Nachteile von Polymer-Reagenzien für die Konjugation an Wirkstoffe des Standes der Technik zumindest teilweise überwunden werden können.

Diese Aufgabe wird erfindungsgemäß durch die Bereitstellung von Verbindungen der Formel (I) gelöst 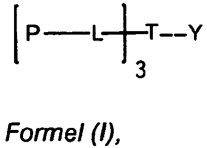 wobei
P und Y jeweils unabhängig voneinander einen Kohlenwasserstoffrest darstellen, welcher Heteroatome enthalten kann, dadurch gekennzeichnet,
   dass P wenigstens eine Gruppe der Formel (IIa)

   R₁-(CH₂-CH₂-O)ₙ-CH₂-CH₂-

   aufweist, worin
R₁ H, OH oder ein Kohlenwasserstoffrest mit 1 bis 50 Kohlenstoffatomen ist, welcher Heteroatome enthalten kann,
n bei jedem Auftreten unabhängig eine ganze Zahl von 2 bis 1000 ist, und dass die Gruppe T die Struktur 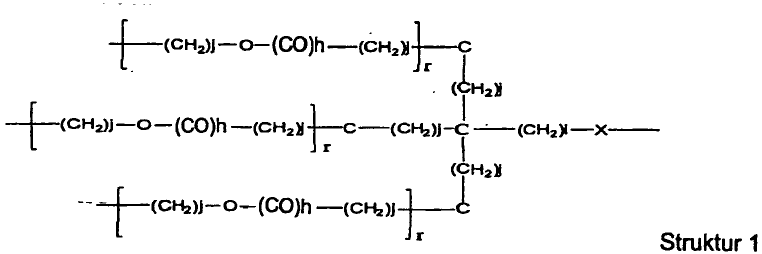 aufweist, wobei
X NH, C=O, S oder O sein kann, h 0 oder 1 ist,
j bei jedem Auftreten unabhängig voneinander eine ganze Zahl
zwischen 0 und 10, und
r bei jedem Auftreten unabhängig voneinander eine ganze Zahl zwischen 0 und 3 ist,
und dass die Gruppe L die Struktur 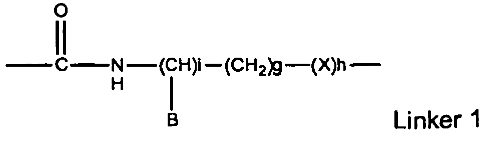 oder 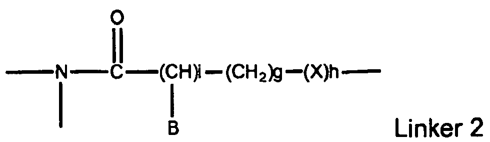 hat, wobei
B H, OH, C₁-C₄-Alkyl, O-R₂, oder CO-R₃ darstellt,
R₂ bei jedem Auftreten unabhängig einen Kohlenwasserstoffrest mit 1 bis 6 C-Atomen darstellt,
R₃ OH oder NR₄R₅ ist,
R₄ und R₅ jeweils unabhängig H oder einen Kohlenwasserstoffrest, welcher Heteroatome enthalten kann, darstellen, wobei R₄ und R₅ zusammen auch ein Ringsystem bilden können,
i und g jeweils eine ganze Zahl zwischen 1 und 10 ist,
X gleich S und
h 0 oder 1 ist,
wobei, wenn L die Struktur Linker 2 hat, P unabhängig voneinander H oder eine Verbindung ist, die eine Gruppe der Formel (IIa) enthält, wobei die Verbindung insgesamt wenigstens drei Gruppen der Formel (IIa) enthält.

Erfindungsgemäß weist die Gruppe T die Struktur 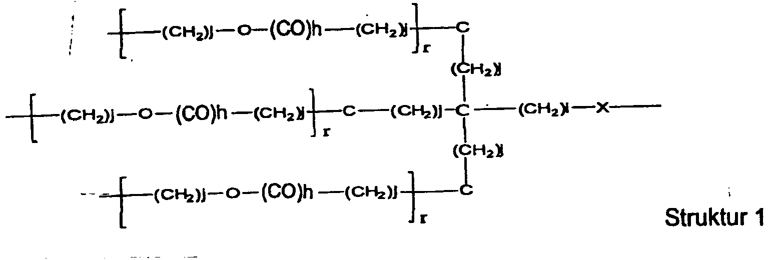 auf. Im Molekül können dann noch weitere Folgeverzweigungen vorliegen.

Die erfindungsgemäßen Verbindungen weisen ein mehrfach verzweigtes Grundgerüst auf, welches unabhängig voneinander durch die Verwendung einer Verzweigungsgruppe T oder/und durch einen verzweigten Linker L oder/und ein verzweigtes Polymer hergestellt werden kann. Die erfindungsgemäßen Verbindungen sind dadurch gekennzeichnet, dass sie mindestens 3, bevorzugt mindestens 4, mehr bevorzugt mindestens 5, und am meisten bevorzugt mindestens 6 Gruppen der Formel (IIa) enthalten. Erfindungsgemäß sind oftmals auch Verbindungen bevorzugt, die mindestens 8, insbesondere mindestens 10 Gruppen der Formel (IIa) aufweisen.

Die Vorteile der hoch verzweigten und monodispersen erfindungsgemäßen Polymer-Reagenzien sind zum Beispiel:
a) Hochmolekulare Polymer-Reagenzien für pharmazeutische Anwendungen mit einem Molekulargewicht von > 30000 Da können nur durch Verzweigung erhalten werden,
b) Monodisperse Polymer-Reagenzien mit pharmazeutisch relevantem Molekulargewicht können nur erhalten werden, indem viele kurze monodisperse Ausgangsmaterialien verzweigt werden,
c) Nur ein hoher Verzweigungsgrad von Polymer-Reagenzien führt zur gewünschten Schutzfunktion bei gleichzeitigem Erhalt der Aktivität des modifizierten Wirkstoffes und
d) Unerwünschte Quervernetzungsreaktionen können vermieden werden.

### Beschreibung der Gruppe Y

Die efindungsgemäßen Verbindungen enthalten als funktionelle Gruppe wenigstens eine Bindegruppe Y, welche eine kovalente Bindung der efindungsgemäßen Verbindung an weitere Moleküle, insbesondere an biotechnologische, pharmazeutische oder synthetische Wirkstoffe, sowie an Oberflächen oder an Biokatalysatoren ermöglicht. Bei der Bindegruppe Y handelt es sich bevorzugt um eine Verbindung, welche mit einer in dem zu koppelnden Wirkstoff vorliegenden funktionellen Gruppe kovalent binden kann, beispielsweise um eine Bindegruppe, die mit einer Aminogruppe, einer Thiolgruppe, einer Carboxylgruppe, einer Guanidingruppe, einer Carbonylgruppe, einer Hydroxylgruppe, einem Heterozyklus, insbesondere mit N als Heteroatom (z.B. in Histidinresten), einer C-nukleophilen Gruppe, einer C-elektrophilen Gruppe, einem Phosphat, einem Sulfat oder ähnlichem bindefähig ist. Möglich sind auch nicht kovalente Bindungen, z.B. Chelate, Komplexe mit Metallen, z.B. an Oberflächen oder mit Radioisotopen sowie Bindungen an Silicium-haltige Oberflächen. Geeignete Bindegruppen sind beispielsweise eine Carbonsäure oder eine aktivierte Carbonsäuregruppe.

Zur späteren Kopplung der Verbindung an ein biotechnologisches oder synthetisches Produkt sowie an Naturstoffe und technische Produkte enthalten die erfindungsgemäßen Verbindungen bevorzugt eine aktivierte Funktionalität Y. In der aktivierten Form ist Y bevorzugt aus der Gruppe ausgewählt die aus (O-alkyl)₂, -OSO₂CH₂CF₃ (Tresyl), (O-aryl)-Azide, -CO-Q, Maleimidyl, -O-CO-Nitrophenyl oder Trichlorphenyl, -S-S-Alkyl, -S-S-Aryl, -SO₂-Alkenyl (Vinylsulfon), -Halogen (Cl, Br, I) besteht, wobei Q unabhängig aus einer Gruppe ausgewählt ist, die aus H, O-Aryl, O-Benzyl, O-N-Succinimid, O-N-Sulfosuccinimid, O-N-Phthalimid, O-N-Glutarimid, O-N-Tetrahydrophthalimid, -N-Norbornene-2,3-dicarboximid, Hydroxybenzotriazole und Hydroxy-7-azabenzotriazole besteht. Bevorzugt handelt es sich bei Y um eine Gruppe -CO-Q. Eine gute Übersicht über mögliche Aktivierungen bietet der Review von Zalipsky, S. erschienen in Bioconjugate Chem. 1995, 6, 150-165.

Durch die Gruppe Y können die erfindungsgemäßen Verbindungen kovalent an Wirkstoffe gebunden werden und somit höchst wünschenswerte, stabile Konjugate bilden.

### Beschreibung der Gruppen P, L und T

Die Reste P, L und T der erfindungsgemäßen Verbindung stellen jeweils unabhängig voneinander Wasserstoff oder einen Kohlenwasserstoffrest dar, welcher gegebenenfalls Heteroatome enthalten kann. Ein Kohlenwasserstoffrest bedeutet hierin, soweit nicht explizit anders angegeben, einen Rest mit 1 bis 100.000 C-Atomen, mehr bevorzugt einen Rest von 1 bis 10.000 C-Atomen, in einigen bevorzugten Fällen 1 bis 50 C-Atomen, welcher 0 bis 10.000, mehr bevorzugt 1 bis 1.000 Heteroatome enthalten kann, beispielsweise ausgewählt aus O, P, N oder S. Die Kohlenwasserstoffreste können linear oder verzweigt sein und gesättigt oder ein- oder mehrfach ungesättigt sein. Ein Kohlenwasserstoffrest kann auch zyklische oder aromatische Abschnitte enthalten. Bevorzugte Kohlenwasserstoffreste sind Alkyl, Cycloalkyl, Alkenyl, Cycloalkenyl, Alkinyl, Cycloalkinyl, Aroyl sowie Heteroaroyl.

Die Gruppe P enthält bei jedem Auftreten vorzugsweise mindestens eine Gruppe der Formel (IIa). In bevorzugten Ausführungsformen ist P verzweigt und enthält zwei oder mehr Gruppen der Formel (IIa).

Durch Verzweigung in der Gruppierung P werden erfindungsgemäß Verbindungen erhalten, welche neben der Kemverzweigung T weitere Folgeverzweigungen aufweisen. Diese Folgeverzweigungen können beispielsweise durch Aminosäuren, durch eine Trisstruktur oder durch ein Ugi-Produkt erhalten werden. Bevorzugte Beispiele für verzweigte Gruppierungen P sind im Folgenden dargestellt.
a) Dies kann zum einen dadurch erreicht werden, dass einfach verzweigte (Y-shaped) Polymer-Reagenzien bei der Synthese verwendet werden. Eine besonders bevorzugte Ausführung auf der Basis einer natürlichen Aminosäure hat die Struktur: 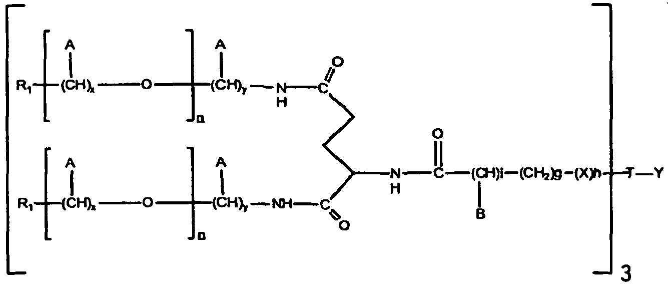 worin
   T die in Formel (I) angegebenen Bedeutungen aufweist,
   Y einen Kohlenwasserstoffrest darstellt, welcher Heteroatome enthalten kann,
   A bei jedem Auftreten unabhängig H, OH, C₁-C₄-Alkyl, O-R₂ oder CO-R₃ darstellt,
   R₁ H, OH oder ein Kohlenwasserstoffrest mit 1 bis 50 Kohlenstoffatomen, insbesondere bis zu 20 Kohlenstoffatomen ist, welcher Heteroatome, insbesondere O, N, P oder/und S enthalten kann,
   R₂ bei jedem Auftreten unabhängig einen Kohlenwasserstoffrest mit 1 bis 6 C-Atomen darstellt,
   R₃ OH, OR₆ oder NR₄R₅ ist,
   R₄, R₅ und R₆ jeweils unabhängig H oder einen Kohlenwasserstoffrest, welcher Heteroatome enthalten kann, darstellen, wobei R₄ und R₅ zusammen auch ein Ringsystem bilden können,
   n bei jedem Auftreten unabhängig eine ganze Zahl von 2 bis 1000, insbesondere von 3 bis 100 und bevorzugt von 4 bis 50 ist und
   x bei jedem Auftreten eine ganze Zahl von 1 bis 10, insbesondere von 2 bis 4 ist und
   y eine ganze Zahl von 0 bis 50, insbesondere von 1 bis 10 darstellt,
   B H, OH, C₁-C₄-Alkyl, O-R₂, oder CO-R₃ darstellt,
   i und g jeweils eine ganze Zahl zwischen 0 und 20, mehr bevorzugt zwischen 1 und 10 und noch mehr bevorzugt 2 und 6,
   X gleich N, S, P, O oder C=O,
   h 0 oder 1 ist,
   wobei die Verbindung insgesamt wenigstens drei Gruppen der Formel (IIa) aufweist, wobei Formel (IIa) die in Formel (I) angegebenen Bedeutungen aufweist.
b) Insbesondere können mehrfach Gruppen der Formel (IIa) eingeführt werden, indem für P eine Verbindung der Formel 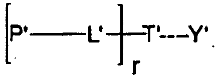 verwendet wird,
   L', T', Y' und P' haben dabei die hierin für L, T, Y und P angegebenen Bedeutungen. In diesem Fall wird z.B. die Synthese mehrfach wiederholt und es werden Dendrimer-artige Strukturen erhalten.
c) Zum anderen kann dies insbesondere durch den Einsatz einer Mehrkomponentenreaktion erreicht werden, z.B. der Ugi-Reaktion. Eine spezielle Ausführungsform ist dementsprechend Formel (III), wobei P = U: 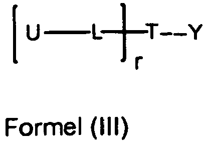 wobei
   U ein Produkt der Ugi-Reaktion der Formel (IV) ist: 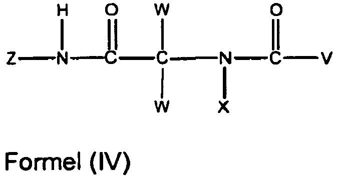
worin
die Reste V, W, X und Z jeweils unabhängig voneinander einen Kohlenwasserstoffrest darstellen, welcher Heteroatome enthalten kann oder/und V, W oder/und X Wasserstoff darstellen.

Die Herstellung und Verwendung einer Gruppe der Formel (III) bzw. (IV) wurde in Patentanmeldung PCT/EP2004/006135 beschrieben.

### Beschreibung der Gruppe L

Die Gruppe L der erfindungsgemäßen Verbindung ist ein Linker der Form 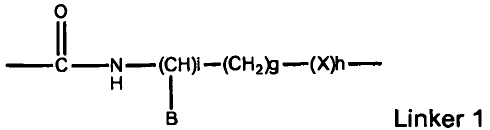 oder 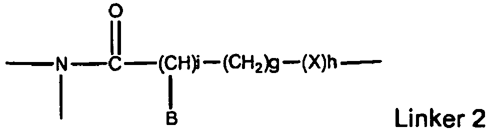 wobei
B H, OH, C₁-C₄-Alkyl, O-R₂, oder CO-R₃ darstellt,
R₂ bei jedem Auftreten unabhängig einen Kohlenwasserstoffrest mit 1 bis 6 C-Atomen darstellt,
R₃ OH oder NR₄R₅ ist,
R₄ und R₅ jeweils unabhängig H oder einen Kohlenwasserstoffrest, welcher Heteroatome enthalten kann, darstellen, wobei R₄ und R₅ zusammen auch ein Ringsystem bilden können,
i und g jeweils eine ganze Zahl zwischen 1 und 10 und mehr bevorzugt 2 und 6, X gleich S,
h 0 oder 1 ist.

Wenn L eine Verzweigung enthält wie bei Linker 2 ausgeführt, dann können entsprechend 2 Polymere der Formel P gebunden sein und dann ist eine bevorzugte Ausführungsform 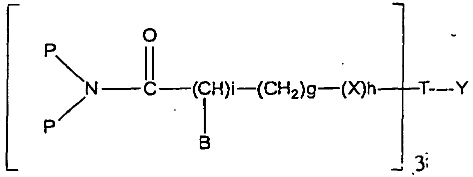
i bei jedem Auftreten 0 oder 1 ist,
g bei jedem Auftreten eine ganze Zahl zwischen 0 und 10, bevorzugt zwischen 0 und 5 ist,
B H, OH, C₁-C₄-Alkyl, O-R₂, oder CO-R₃ darstellt,
R₁ H, OH oder ein Kohlenwasserstoffrest mit 1 bis 50 Kohlenstoffatomen ist, welcher Heteroatome enthalten kann,
R₂ bei jedem Auftreten unabhängig einen Kohlenwasserstoffrest mit 1 bis 6 C-Atomen darstellt,
R₃ OH oder NR₄R₅ ist,
R₄ und R₅ jeweils unabhängig H oder einen Kohlenwasserstoffrest, welcher Heteroatome enthalten kann, darstellen, wobei R₄ und R₅ zusammen auch ein Ringsystem bilden können,
P und P' unabhängig voneinander H oder Verbindungen sind, die Gruppe der Formel II enthalten.
X gleich NR₁, S, O oder C=O,
h 0 oder 1 ist.

### Beschreibung Gruppe T

Die Verzweigungsgruppe T ist repräsentiert durch eine Alkylkette, die verzweigt, gesättigt ist, und die Heteroatome N und O, z.B. zwischen der Verzweigung und T enthalten kann und die Struktur 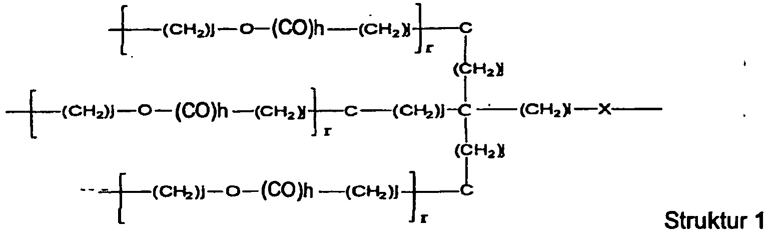 hat, wobei
- X: NH, C=O, S oder O sein kann, h 0 oder 1 ist,
- j: bei jedem Auftreten unabhängig voneinander eine ganze Zahl zwischen 0 und 10, und
- r: bei jedem Auftreten unabhängig voneinander eine ganze Zahl zwischen 0 und 3 ist.

In solchen Verbindungen fungiert Tris als Verzweigungskomponente. Die Verbindungen weisen als wenigstens eine Gruppe T eine Tris-Struktur auf.

Die Erfindung stellt hoch verzweigte Reagenzien bereit, welche mehrere und insbesondere eine große Anzahl an Gruppierungen der Formel (IIa) enthält, welche einem Konjugat aus Verbindung der Formel (I) und einem Wirkstoff insbesondere eine verminderte Immunogenität, eine verlängerte Halbwertszeit im Körper, eine höhere Proteolysestabilität, eine Erhöhung der Löslichkeit, eine Verringerung der Toxizität, eine verbesserte pH-Stabilität sowie eine erhöhte Thermostabilität verleihen.

Insbesondere ist es erfindungsgemäß möglich, eine gute Abschirmung durch eine oder mehrere kurzkettige Gruppen der Formel (IIa) zu erreichen, wobei kurzkettige Gruppen der Formel (IIa) (bevorzugt mit n ≤ 50, insbesondere n ≤ 25) mit gleicher Kettenlänge mit guter Reproduzierbarkeit erhalten und eingeführt werden können. Alternativ ist es auch möglich, gleichzeitig Gruppen der Formel (IIa) mit unterschiedlichen Kettenlängen einzuführen. Ferner ist es auch möglich, polydisperse Gruppierungen der Formel (IIa) einzusetzen.

Die Gruppen der Formel (IIa) stellen das Polyalkylenoxid Polyethylenglykol dar.

Bei dem Rest R₁ handelt es sich um Wasserstoff, Hydroxy oder einen Kohlenwasserstoffrest mit 1 bis 50 Kohlenstoffatomen, mehr bevorzugt 1 bis 30 Kohlenstoffatomen und am meisten bevorzugt 1 bis 10 Kohlenstoffatomen, welcher gegebenenfalls Heteroatome enthalten kann. Der Rest R₁ kann gesättigt oder ein- oder mehrfach ungesättigt sowie linear, verzweigt oder zyklisch sein. Besonders bevorzugt ist R₁ HO, CH₃-O, CH₃-(CH₂)ₐ-O, (CH₃)₂CH-O, wobei a eine ganze Zahl zwischen 1 und 20 ist. R₁ kann weiterhin bevorzugt ausgewählt werden aus einem Acetal, z.B. (CH₃O)₂- und (CH₃-CH₂O)₂-, einem Aldehyd, z.B. OHC-CH₂-O-, einer Alkenylgruppe, z.B. CH₂=CH-CH₂-O-, einem Acrylat, z.B. CH₂=CH-CO₂-, oder einem Methacrylat, z.B. CH₂=C(CH₃)-CO₂-, einem Acrylamid, z.B. CH₂=CH-CONH-, einer Aminoalkylgruppe, z.B. H₂N-CH₂-CH₂-, einer geschützten Aminoalkylgruppe, z.B. **A**--NH-CH₂-CH₂--, wobei **A** eine Schutzgruppe darstellt, insbesondere N-Acyl, N-Sulfonyl, N-Silylschutzgruppen, wie z.B. tert. Boc-, Alloc-, Fmoc, Tr-, Z-, Moz-, einer Thioalkylgruppe HS-CH₂-CH₂--, oder einer geschützten Thioalkylgruppe.

In speziellen Ausführungsformen kann der Rest R₁ auch eine aktivierte Funktionalität sein. Dann ist R₁ bevorzugt aus der Gruppe ausgewählt, die aus -OSO₂CH₂CAF₃ (Tresyl), -Azide, speziell (O-aryl)-Azide, -CO-Q, Maleimidyl, -O-CO-Nitrophenyl oder Trichlorphenyl, -S-S-Alkyl, -S-S-Aryl, -SO₂-Alkenyl (Vinylsulfon), -Halogen (Cl, Br, I) besteht, wobei Q unabhängig aus einer Gruppe ausgewählt ist, die aus H, O-Aryl, O-Benzyl, O-N-Succinimid, O-N-Sulfosuccinimid, O-N-Phthalimid, O-N-Glutarimid, O-N-Tetrahydrophthalimid,-N-Norbornene-2,3-dicarboximid, Hydroxybenzotriazole und Hydroxy-7-azabenzotriazole besteht.

Erfindungsgemäß ist die Gruppierung der Formel (IIa)

*R₁-(CH₂-CH₂-O)ₙ-CH₂-CH₂-* *Formel (IIa),*

wobei n bei jedem Auftreten unabhängig eine ganze Zahl von 2 bis 1000 ist.

n der erfindungsgemäßen Formel IIa ist bei jedem Auftreten unabhängig eine ganze Zahl von 2 bis 1000, mehr bevorzugt bis 500, noch mehr bevorzugt bis 250, insbesondere mindestens 3 und am meisten bevorzugt mindestens 4 bis 50. Efindungsgemäß ist es möglich, Verbindungen mit einer großen Anzahl an Gruppierungen der Formel (IIa) bereitzustellen, mit mindestens 2, insbesondere mindestens 3, bevorzugt mindestens 4, mehr bevorzugt mindestens 5 und am meisten bevorzugt mindestens 9 Gruppierungen der Formel (IIa). Oftmals sind jedoch bereits Verbindungen besonders bevorzugt, welche 3 oder 4 Gruppierungen der Formel (IIa) enthalten.

Die vorliegende Erfindung trägt dazu bei, die beschriebenen, im Stand der Technik auftretenden Nachteile oder Einschränkungen zu verringern. Sie umfasst die Synthese mehrfach verzweigter Verbindungen, welche zur Modifikation von Naturstoffen, technischen Produkten, biotechnologischen und synthetischen Produkten oder von pharmazeutischen Wirkstoffen verwendet werden können.

Die erfindungsgemäßen Verbindungen enthalten eine aktivierte Linker-gruppe, die mit einer oder mehreren Aminofunktionalitäten oder anderen funktionellen Gruppen eines biotechnologischen oder synthetischen Produktes im Rahmen einer chemischen Reaktion unter milden Reaktionsbedingungen eine kovalente Bindung eingeht und mindestens eine Polymerfunktion, die die biochemischen und pharmakologischen Eigenschaften des Konjugats beeinflussen.

Die vorliegende Erfindung stellt bevorzugt eine mehrfach verzweigte Struktur bereit, sowie deren Synthese und Anwendung zur Modifikation von biotechnologischen Produkten. Zur Erhöhungen des Verzweigungsgrades können zusätzlich verzweigte Gruppen der Formel P eingesetzt werden. Diese können zum Beispiel durch die Verwendung von Verbindungen der Formel [P'-L'-]-T'---Y' als P oder durch Verwendung von Produkten aus einer Mehrkomponenten-Reaktion, z.B. der Ugi-Reaktion, erreicht werden.

Die vorliegende Erfindung stellt bevorzugt eine verzweigte Polymerverbindung bereit die nur eine einzige aktivierte Linkergruppe trägt, wodurch Quervernetzungsreaktionen vermieden werden. Diese Polymerverbindung ist hydrophil und biologische verträglich. Sie ist einfach herzustellen und eröffnet breite Anwendungsmöglichkeiten bei der Modifikation pharmazeutischer Wirkstoffe und technisch eingesetzter Produkte. Konjugate der erfindungsgemäßen Polymerverbindung mit pharmazeutischen Wirkstoffen ermöglichen eine Verbesserung des therapeutischen Einsatzes. Weiterhin ermöglichen diese Konjugate bei Verlängerung der Wirkdauer die Verringerung der zu verabreichenden Menge an Wirkstoff, so zum Beispiel für die Behandlung von Krebs- und Infektionskrankheiten.

In speziellen Ausführungsformen enthält die erfindungsgemäße Polymerverbindung mehrere aktivierte Linkergruppen. In diesen Fällen wird die Kopplung mehrerer Wirkstoffmoleküle an das zentrale Polymergerüst ermöglicht. Auf diese Weise können z.B. Multivalenzeffekte erreicht werden, die zu einer verbesserten biologischen Aktivität eines Wirkstoffes führen, während gleichzeitig die Halbwertszeit des Moleküls verlängert wird.

Die Anmeldung offenbart weiterhin ein Verfahren zur Herstellung der erfindungsgemäßen Verbindungen, wobei man in einer mehrstufigen Synthese aufbauend auf einer verzweigten Komponente T ein mehrfach verzweigtes Polymer-Reagenz herstellt. Insbesondere ist es möglich, für die Synthese eine radikalische Addition eines Thiols an eine Doppelbindung einzusetzen. Alternativ dazu kann die Michael Addition unter Verwendung von Acrylsäurederivaten zur Anwendung kommen.

Im Rahmen der vorliegenden Erfindung werden auch Konjugate der bifunktionalen, verzweigten Polymerverbindung mit biologisch aktiven Substanzen, wie Proteinen (z.B. humane Wachstumsfaktoren), Enzymen, Co-Faktoren für Enzyme (z.B. NAD+/NADH), Liposomen, Antikörpern, synthetischen, kleinen Wirkstoffen, Phospholipiden, Lipiden, Nucleosiden, Oligonucleotiden, Mikroorganismen, humanen Zellen und Oberflächen bereitgestellt.

Die Erfindung betrifft deshalb auch Konjugate, umfassend Verbindungen der Formel (I) in kovalenter Verknüpfung an weitere Moleküle, insbesondere an Wirkstoffe, wie etwa Biopharmazeutika oder synthetische Wirkstoffe, oder biotechnologische Substanzen, die im Bereich "Life Science" eingesetzt werden, z.B. im Bereich Proteomics oder Diagnostik. Solche Substanzen sind z.B. Enzyme, insbesondere Proteasen, wie z.B. Trypsin oder Chymotrypsin. Die in den Konjugaten an den erfindungsgemäßen Verbindungen verknüpften Verbindungen sind vorzugsweise Biopharmazeutika, peptidische Wirkstoffe oder andere biologisch aktive Substanzen. Weiterhin können auch Konjugate mit Oberflächen oder Biokatalysatoren gebildet werden.

Die Erfindung betrifft weiterhin eine pharmazeutische Zusammensetzung, umfassend die erfindungsgemäßen Verbindungen und insbesondere die erfindungsgemäßen Konjugate. Solche pharmazeutischen Zusammensetzungen können beispielsweise zur Prävention oder Behandlung von Krebs, Herz-Kreislauf-Erkrankungen, Stoffwechsel-Erkrankungen, neuronalen bzw. cerebralen Erkrankungen oder entzündlichen Prozessen, wie Infektionen, Immunerkrankungen oder Autoimmunerkrankungen (z.B. rheumatoide Arthritis) eingesetzt werden.

Die erfindungsgemäßen Verbindungen bzw. Konjugate eignen sich auch hervorragend als diagnostische Mittel.

Die Erfindung wird durch die beigefügten Abbildungen und die nachfolgenden Beispiele weiter erläutert.
Abbildung 1: Synthese der Tricarbonsäure in einer Michael-Addition.

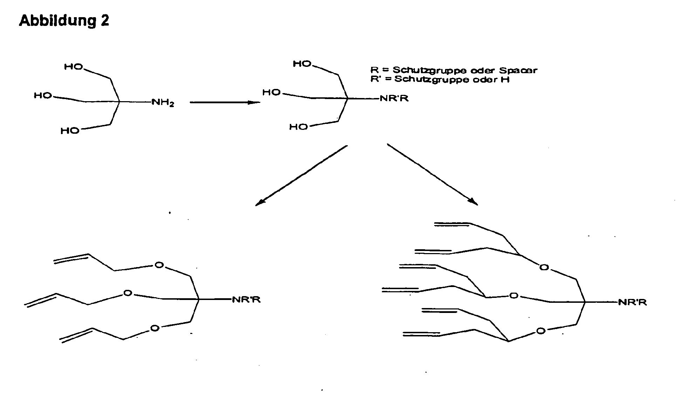

Abbildung 2: Einführung olefinischer Gruppen.

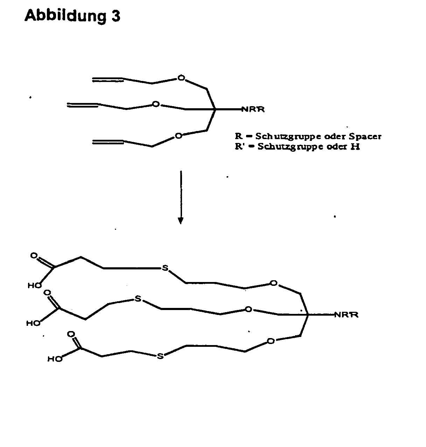

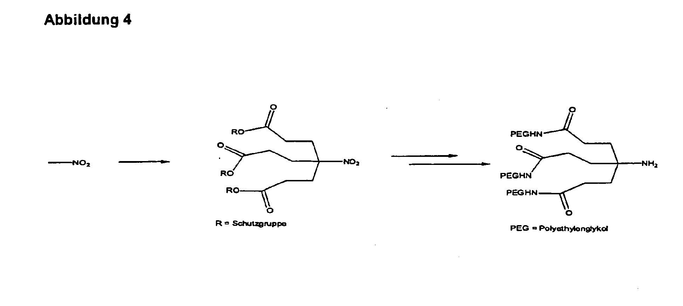

Abbildung 3: Radikalische Addition unter Bildung eines Thioethers. Vergleichsabbildung 4: Michael-Addition an Nitromethan.
Abbildung 5: Radikalische Addition von 2-Mercaptoethylamin.
Abbildung 6: Dreifache Verzweigung auf der Basis des Zentralbausteins.
Abbildung 7: Sechsfache Verzweigung durch Einbau eines Y-förmigen PEGs.
Abbildung 8: Neunfache Verzweigung durch Wiederholung des Zentralbausteins.
Abbildung 9: Tricarbonsäure-Zentralbaustein mit geschützter Carboxylfunktion.

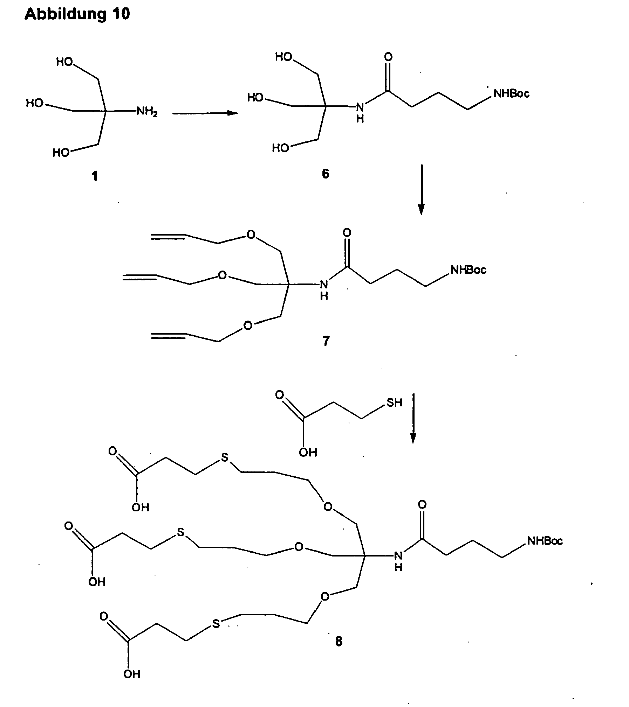

Abbildung 10: Tricarbonsäure-Zentralbaustein mit geschützter Aminofunktion.
Abbildung 11: 6-arm PEG mit *tert*-Butyl-geschützter Carboxylfunktion.
Abbildung 12: 6-arm PEG mit *Boc*-geschützter Aminofunktion.
Abbildung 13: 3-arm PEG mit Boc-geschützter Aminofunktion.
Abbildung 14: 6-arm PEG mit *tert*-Butyl-geschützter Carboxylfunktion und Ugi-Motiv als Folgeverzweigung.
Abbildung 15: 6-arm PEG mit freier Carbonsäure.
Abbildung 16: 6-arm PEG mit freiem Amin.
Abbildung 17: 3-arm PEG mit freiem Amin.
Abbildung 18: 6-arm PEG.
Abbildung 19: 6-arm PEG mit Glutarsäure-Linker NHS-aktiviert.
Abbildung 20: 6-arm PEG NHS Aktivester mit Ugi-Motiv.
Abbildung 20a: 6-arm PEG NHS-Aktivester mit Glutaminsäureverzweigung.
Abbildung 21: 9-arm PEG; Folgeverzweigung durch Verwendung des Zentralbausteins.
Abbildung 22: Aldehyd-Aktivierung zur reduktiven Aminierung von Aminogruppen.
Abbildung 23: Beispiele für Bindegruppe Y.
Abbildung 24: Schema zur Bildung einer bevorzugten Verzweigungskomponente.

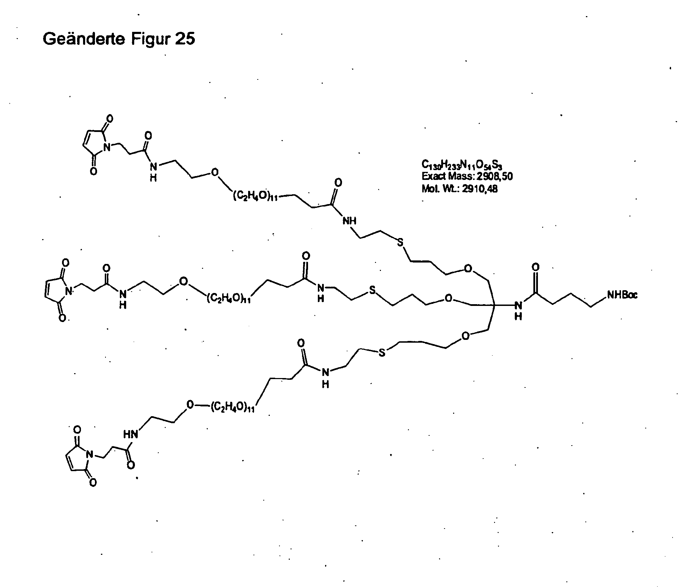

Abbildung 25: 3-arm PEG mit drei Maleimidgruppen, T mit Boc-geschützter Aminofunktion.

### A. Beispiele für erfindungsgemäße Verbindungen

Für die Herstellung der zentralen Verzweigungsgruppe T einschließlich der Bindegruppe Y steht eine Vielzahl von synthetischen Möglichkeiten zur Verfügung. Eine Möglichkeit besteht darin, diesen Baustein aus dem bifunktionalen Trishydroxyaminomethan (TRIS) aufzubauen. Im Hinblick auf die wirtschaftliche Darstellung von hoch verzweigten PEGylierungsreagenzien ist es vorteilhaft, solche funktionellen Gruppen in das Zentralmolekül einzubauen, die sich später in guten Ausbeuten umsetzen lassen. Eine Modifikation der Hydroxygruppen des TRIS-Bausteins führt so zu einer bifunktionalen Tricarbonsäure. Zuvor wird jedoch die Aminogruppe von TRIS mit einer Schutzgruppe (z.B. Boc, Bis-Benzyl) oder einem Spacer geschützt.

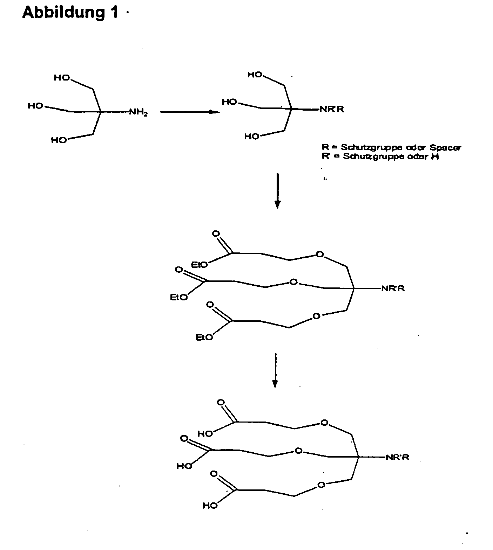

Das Syntheseschema in Abbildung 1 zeigt die direkte Modifikation der Hydroxygruppen mit einem Acrylsäureester im Rahmen einer Michael-Addition. Die Carboxylfunktion kann auch über eine radikalische Addition einer Thiocarbonsäure an ein zuvor synthetisiertes Olefin bzw. Polyolefin eingeführt werden (siehe Abbilding 2 und 3).

Eine nicht erfindungsgemäße alternative Möglichkeit zur Darstellung einer multifunktionalen Tricarbonsäure unabhängig von TRIS als Startmaterial geht von der CH-aciden Verbindung Nitromethan aus. Hierbei wird Nitromethan in einer Michael-Adddition unter Verwendung einer starken Base mit einem Acrylsäurederivat umgesetzt. Später kann die Nitro-Gruppe zur Aminofunktion reduziert werden (siehe Vergleichsabbildung 4).

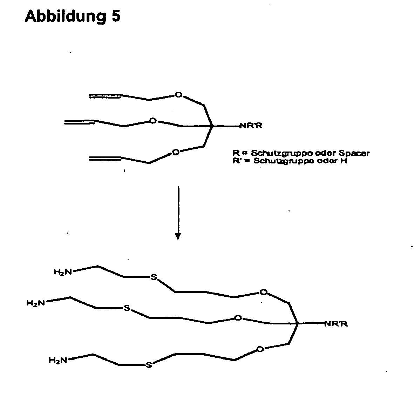

Ausgehend von der dreifach-verzweigten Zentralstruktur lassen sich auch andere funktionelle Gruppen einführen. So kann das Konzept der radikalischen Addition an Olefine auch zur Einführung von Aminogruppen dienen, wie dies in Abbildung 5 dargestellt wird.

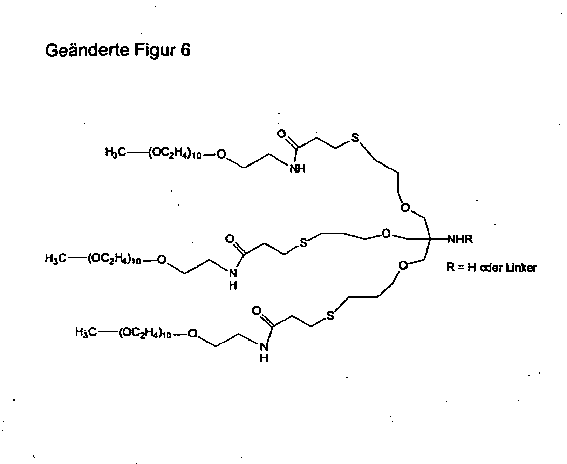

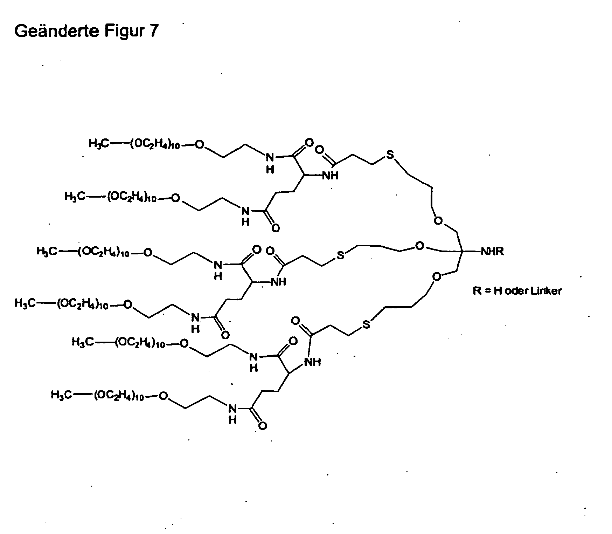

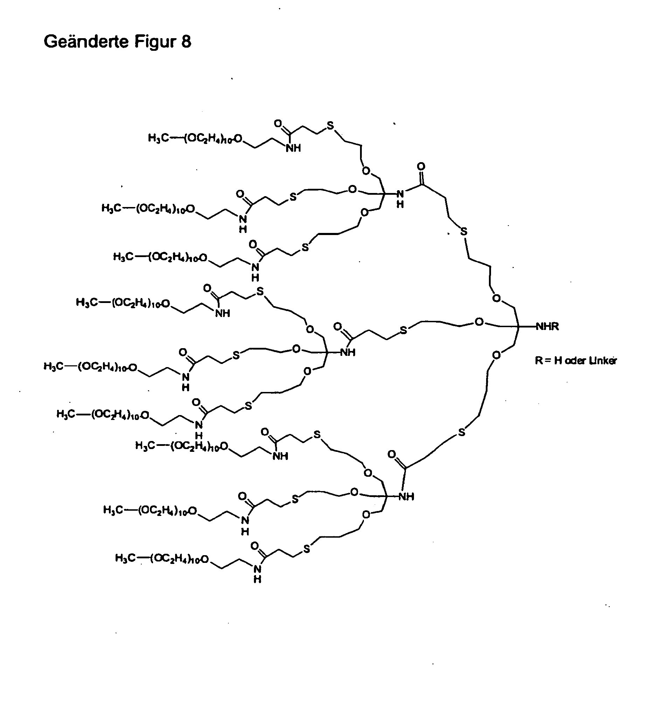

Auf der Grundlage der verschiedenen, zentralen Verzweigungsbausteine können die verschiedensten, mehrfach verzweigten PEGylierungs-Reagenzien aufgebaut werden. Im einfachsten Fall werden lineare PEG-Ketten über eine Amidierung, reduktive Aminierung oder radikalische Addition direkt gekoppelt, sodass eine dreifache Verzweigung entsteht. Weiterhin kann eine Folgeverzweigung durch Kopplung bereits verzweigter PEGs generiert werden. Dies können a) die bereits bekannten Y-förmigen PEGs sein, die auf der Basis von natürlichen Aminosäuren hergestellt werden, b) sekundäre Amine, die zwei PEG-Ketten tragen, verzweigte PEGs die aus einer Ugi-Reaktion entstehen oder c) der dreifach verzweigte Baustein wird wiederholt zur Kopplung an den Grundbaustein genutzt. Die Bausteine können direkt oder über Spacer gekoppelt sein. In den folgenden drei Abbildungen sind Beispiele für diese Möglichkeiten dargestellt (siehe Abb. 6 bis 8).

### 3. Ausführungsbeispiele bevorzugter PEG-Reagenzstrukturen:

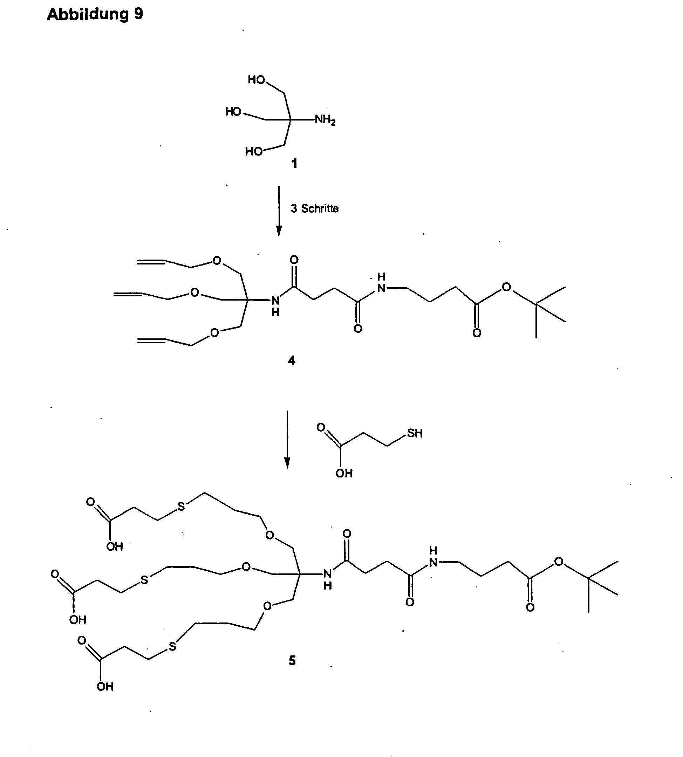

### a) Beispiele für die Herstellung des Zentralbausteins auf TRIS-Basis (siehe Abb. 9)

### Herstellverfahren von 5

Zu einer Lösung aus TRIS (3,97 g; 32,8 mmol), Mono-*tert*-butylsuccinat (5,71 g; 32,8 mmol) und NHS (0,4 g; 3,3 mmol) in DMF (100 mL) wird bei 20 bis 25°C. DCC (7,4 g; 36,0 mmol) gegeben. Anschließend wird das Reaktionsgemisch 24 Stunden bei 20 bis 25°C gerührt. Danach wird der gebildete Dicyclohexylharnstoff über einen Filter entfernt und das Filtrat im Vakuum eingeengt (50 mbar; 60 °C). Eine Plug-Filtration über Kieselgel (Dichlormethan:Methanol; 80:20) und anschließende Kristallisation aus TBME ergibt 2 (4,5 g; 50 %) als weißen, kristallinen Feststoff.
¹H-NMR (300 MHz, CDCl₃): δ = 1,40 (9H); 2,39 (4H); 3,52 (6H); 4,70 (3H); 7,2 (1 H).
¹³C-NMR (75 MHz, CDCl₃): δ = 27,75; 30,50; 30,73; 60,60; 62,29; 79,59; 171,63; 172,27.
LC-MS: m/z 278,1 [M+H]⁺; C₁₂H₂₃NO₆.

Eine Suspension aus NaH (3,3 g; 60% in ÖI) in THF (30 mL) wird bei 0 bis 5°C zu einer Lösung aus **2** (4,5 g; 16,3 mmol) und Allylbromid (5,4 mL; 62 mmol) in THF (120 mL) gegeben. Die dabei gebildete zähe Suspension wird anschließend 4 Stunden bei 0 bis 5°C und danach bei 20 bis 25°C gerührt. Die Reaktion wird anschließend durch Zugabe von 0,1 M HCl abgebrochen und gleichzeitig ein pH-Wert von 6-7 eingestellt. Danach wird das THF im Vakuum entfernt und der Rückstand in Ethylacetat (150 mL) aufgenommen. Diese Lösung wird mit 0,5 M NaOH (100 mL) gewaschen. Nach der Phasentrennung wird die wässrige Phase mit einer 1 M HCl auf pH 2-3 eingestellt und zweimal mit Dichlormethan (je 100 mL) extrahiert. Die vereinigten organischen Phasen werden im Vakuum eingeengt. Säulenchromatographische Reinigung des öligen Rückstands and Kieselgel (Dichlormethan:Methanol; 90:10) ergibt **3** (4,8 g; 85%) als farbloses, zähes ÖI.
¹H-NMR (300 MHz, CDCl₃): δ = 2,37 (4H); 3,63 (6H); 3,95 (6H); 5,05-5,35 (6H); 5,75-6,00 (3H); 7,29 (1 H); 12,04 (1 H).
¹³C-NMR (75 MHz, CDCl₃): δ = 29,15; 30,55; 59,70; 67,86; 71,46; 116,21; 135,35; 171,29; 173,87.
C₁₇H₂₇NO₆.

Zu einer Lösung aus **3** (4,7 g; 13,8 mmol), dem Hydrochlorid von 4-Aminobuttersäure-*tert*-butylester (2,8 g; 14,3 mmol), Triethylamin (2,1 mL) und NHS (0,3 mg; 2,7 mmol) in Dichlormethan (100 mL) wird DCC (3,7 g; 17,9 mmol) bei 20 bis 25°C gegeben. Das Reaktionsgemisch wird anschließend 24 Stunden bei 20 bis 25°C gerührt. Danach wird der Dicyclohexylharnstoff über einen Filter entfernt, das Filtrat im Vakuum eingeengt und der Rückstand über eine Plug-Filtration an Kieselgel gereinigt. **4** (4 g; 60%) wird als weißer Feststoff erhalten.
¹H-NMR (200 MHz, DMSO-d6): δ = 1,39 (9H); 1,56-1,62 (2H); 2,10-2,35 (6H); 3,00-3,06 (2H); 3,59 (6H); 3,91 (6H); 5,05-5,29 (6H); 5,75-5,98 (3H); 7,23 (1 H); 7,80 (1 H).
¹³C-NMR (50 MHz, DMSO-d6): δ = 24,73; 27,75; 30,91; 31,45; 32,23; 37,78; 59,63; 67,83; 71,46; 79,48; 116,18; 135,31; 171,33; 171,71; 172,02.
LC-MS: m/z 483,6 [M+H]⁺; 505,6 [M+Na]⁺; C₂₅H₄₂N₂O₇.

3-Mercaptopropionsäure (4,4 mL; 41 mmol) wird zu einer Lösung aus **4** (2,5 g; 5,2 mmol) in Toluol (45 mL) gespritzt. Zu dieser Reaktionsmischung wird AIBN (108 mg) gegeben und die resultierende Lösung wird anschließend auf 75 bis 80°C erwärmt. Nach einer Reaktionszeit von 1,5 Stunden bei 75 bis 80°C wird das Reaktionsgemisch im Vakuum eingeengt. Die chromatographische Reinigung des Rückstands an Kieselgel (Dichlormethan:iso-Propanol; 90:10) ergibt den Zentralbaustein 5 (3,4 g; 80 %) als zähes, farbloses ÖI.
¹H-NMR (200 MHz, DMSO-d6): δ = 1,39 (9H); 1,54-1,80 (8H); 2,12-2,35 (6H); 2,40-2,80 (18H); 2,96-3,08 (2H); 3,35-3,50 (6H) 3,54 (6H); 7,20 (1 H); 7,80 (1 H).
¹³C-NMR (50 MHz, DMSO-d6): δ = 24,74; 26,36; 27,78; 29,22; 30,88; 31,46; 32,24; 34,46; 37,82; 59,65; 68,11; 69,17; 79,50; 171,39; 171,67; 172,05; 173,02.
LC-MS: m/z 801,7 [M+H]⁺; C₃₄H₆₀N₂O₁₃S₃.

### Herstellverfahren von 8

Zu einer Lösung aus TRIS (11,9 g; 98,4 mmol), Boc-γ-Aminobuttersäure (20 g; 98,4 mmol) und NHS (1,1 g; 9,8 mmol) in DMF (400 mL) wird bei 20 bis 25°C DCC (21,3 g; 103,0 mmol) gegeben. Anschließend wird das Reaktionsgemisch 24 Stunden bei 20 bis 25°C gerührt. Danach wird die Reaktionsmischung über eine Glasfritte filtriert und das Filtrat im Vakuum eingeengt (50 mbar; 60 °C). Anschließend wird der breiige Rückstand über einen Kieselgel-Plug (Dichlormethan:Methanol; 80:20) filtriert, das Filtrat nochmals eingeengt und dieser Rückstand aus Ethylacetat/Methylcyclohexan (70:30) kristallisiert. Die Kristallisation ergibt 6 (24,8 g; 82 %) als weißen, kristallinen Feststoff.

In einer Lösung aus 6 (10 g; 32,6 mmol), Allybromid (9,1 mL; 104 mmol) und Toluol (200 mL) wird der Phasentransferkatalysator Tetrabutylammoniumbromid (1g) suspendiert. Zu dieser Suspension wird bei 20 bis 25°C NaOH (100 mL; 30%) gegeben. Das gebildete Zweiphasengemisch wird anschließend bei 20 bis 25°C 4 Tage gerührt. Danach wird das Reaktionsgemisch mit Wasser (150 mL) verdünnt. Diese Mischung wird nachfolgend mit Ethylacetat extrahiert und der organische Extrakt wird im Vakuum eingeengt. Die chromatographische Reinigung des öligen Rückstands an Kieselgel (Ethylacetat:Methylcyclohexan; 50:50) ergibt 7 (6,5 g; 46%) als farbloses, zähes ÖI.
¹H-NMR (300 MHz, DMSO-d6): δ = 1,37 (9H);1,51-1,60 (2H); 2,0-2,08 (2H); 2,82-2,95 (2H); 3,60 (6H); 3,92 (d, 6H) 5,08-5,27 (6H); 5,75-5,92 (3H); 6,74 (t, NH, 1H); 7,17 (NH, 1H).
¹³C-NMR (75,4 MHz, DMSO-d6): δ = 26,06; 28,24; 33,44; 39,32; 59,65; 67,79; 71,44; 77,40; 116,15; 135,26; 155,59; 172,23.
LC-MS: m/z 427,27 [M+H]⁺; 449,25 [M+Na]⁺; C₂₂H₃₈N₂O₆.

3-Mercaptopropionsäure (2,8 mL; 31,8 mmol) wird zu einer Lösung aus **7** (1,7 g; 4,0 mmol) in Toluol (40 mL) gespritzt. Zu dieser Reaktionsmischung wird AIBN (83 mg) gegeben und die resultierende Lösung wird anschließend auf 75 bis 80°C erwärmt. Danach wird das Reaktionsgemisch 1,5 Stunden bei 75 bis 80°C gerührt und anschließend im Vakuum eingeengt. Die chromatographische Reinigung des Rückstands an Kieselgel (Dichlormethan:iso-Propanol; 90:10) ergibt den Zentralbaustein **8** (2,5 g; 82 %) als zähes, farbloses ÖI.
¹H-NMR (300 MHz, DMSO-d6): δ = 1,35 (9H);1,47-1,58 (2H); 1,63-1,78 (6H); 1,98-2,07 (2H); 2,40-2,56 (12H); 2,59-2,66 (6H); 2,83-2,92 (2H); 3,39-3,43 (6H); 3,53 (s, 6H); 6,71 (1 H); 7,07 (1 H); 12,15 (3H).
¹³C-NMR (75,4 MHz, DMSO-d6): δ = 26,07; 26,37; 27,76; 28,26; 29,23; 33,46; 34,46; 38,77; 59,66; 68,02; 69,15; 77,43; 155,59; 172,16; 172,98.
LC-MS: m/z 745,1 [M+H]⁺; 783,1 [M+K]⁺; C₃₁H₅₆N₂O₁₂S₃.

### b) Beispiele für die Herstellung hoch verzweigter PEG-Reagenzien

### Allgemeines Verfahren für die Amidierung des TRIS-basierten Zentralbausteins mit linearen oder verzweigten PEG-Aminen am Beispiel von 9

Zu einer Lösung aus der Tricarbonsäure **5** (3,3 g; 4,1 mmol), dem Y-förmigen PEG-Amin (14,1 g; 12,3 mmol) und NHS (290 mg; 2,5 mmol) in Dichlormethan (100 mL) wird bei 20 bis 25°C DCC (2,5 g; 12,3 mmol) gegeben. Anschließend wird das Reaktionsgemisch 24 Stunden bei 20 bis 25°C gerührt. Danach wird die Reaktionsmischung über eine Glasfritte filtriert und das Filtrat im Vakuum eingeengt. **9** (15,7 g; 90 %) wird ohne weitere Reinigungsschritte als farbloses, zähes Öl erhalten. Bei Bedarf kann das Produkt auch säulenchromatographisch an Kieselgel (Dichlormethan/Methanol; 85:15) weiter aufgereinigt werden. Diese allgemeine Herstellungsvorschrift ist für alle weiteren Ausführungsbeispiele gültig.
MALDI-MS: m/z 4194,4 [M+Na]⁺; C₁₈₇H₃₆₃N₁₁O₈₂S₃.

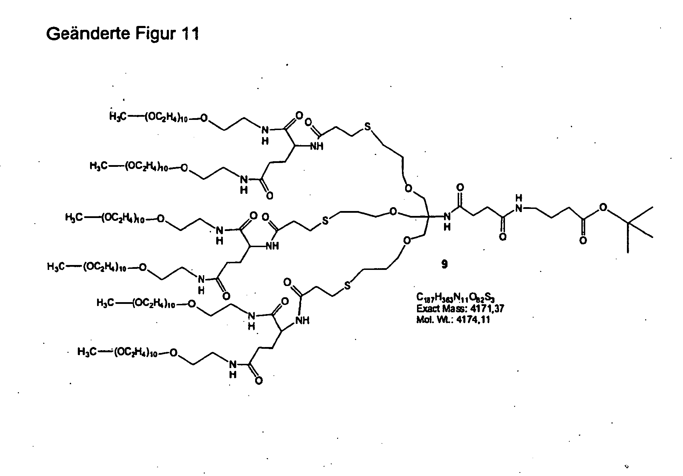

Abbildung 11: 6-arm PEG mit tert-Butyl-geschützter Carboxylfunktion

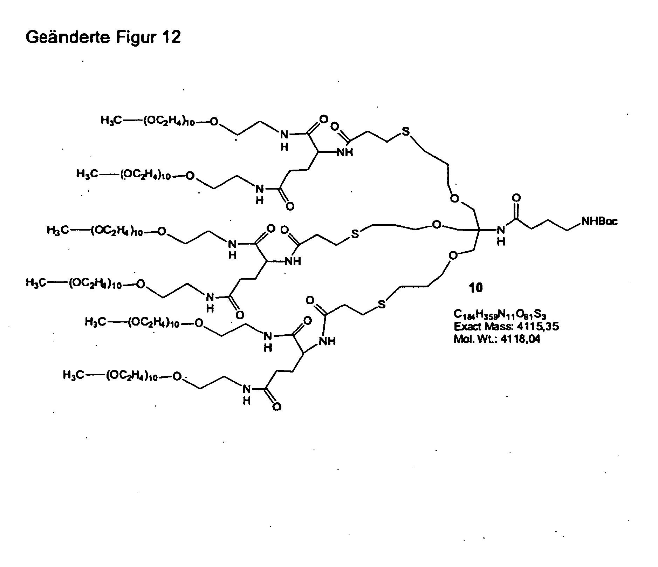

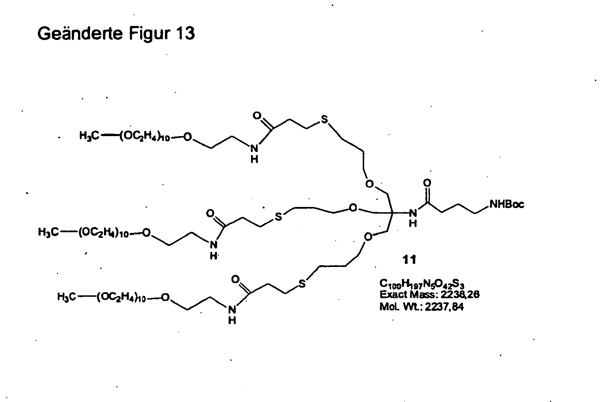

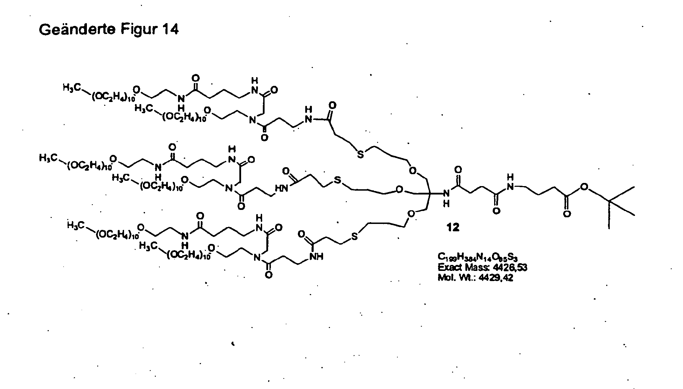

**Die Abbildungen 12 bis 14** zeigen weitere Ausführungsbeispiele für die Amidierung:
MALDI-MS: m/z 4138,4 [M+Na]⁺; C₁₈₄H₃₅₉N₁₁O₈₁S₃ **(****Abb. 12****)**
MALDI-MS: m/z 2259,4 [M+Na]⁺; C₁₀₀H₁₉₇N₅O₄₂S₃ **(****Abb. 13****)**
MALDI-MS: m/z 4449 [M+Na]⁺; 4465,3 [M+K]⁺; C₁₉₉H₃₈₄N₁₄O₈₅S₃(Abb.14)

### Entfernung der Schutzgruppen Boc und tert-Butyl

Die Boc-Schutzgruppe der Aminofunktion sowie die tert-Butyl-Schutzgruppe der Carboxylfunktion werden standardmäßig unter Verwendung von HCl (4M in Dioxan) in Dichlormethan gespalten. Die Reaktionsprodukte können durch wässrige Extraktion gereinigt werden und werden als Rohprodukte weiterverwendet. Es werden Ausbeuten zwischen 50 bis 85% erhalten.

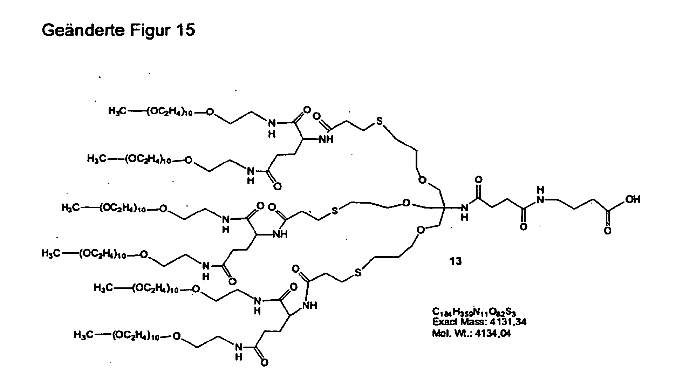

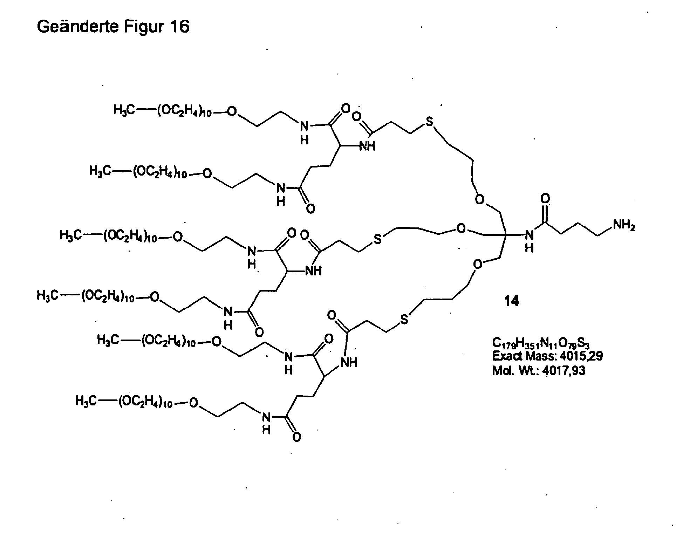

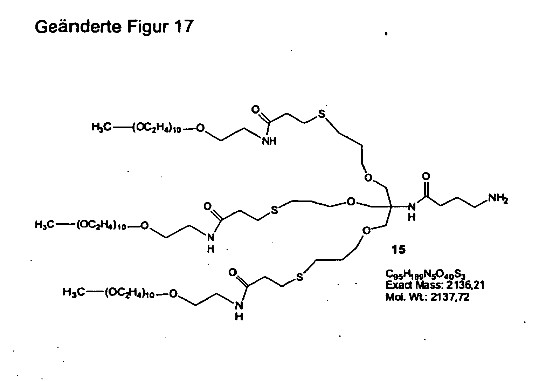

**Die Abbildungen 15 bis 17** zeigen Ausführungsbeispiele für die Deblockierung:
MALDI-MS: m/z 4154 [M+Na]⁺; C₁₈₄H₃₅₉N₁₁O₈₂S₃ **(****Abb.** 15).
MALDI-MS: m/z 4016,35 [M+H]⁺; 4038,3 [M+Na]⁺; 4054,3 [M+K]⁺; C₁₇₉H₃₅₁N₁₁O₇₉S₃ **(****Abb. 16****).**
MALDI-MS: m/z 2137,3 [M+H]⁺; C₉₅H₁₈₉N₅O₄₀S₃**(****Abb. 17****).**

### Allgemeines Verfahren für die Herstellung von NHS-Aktivestern am Beispiel von 16

Zu einer Lösung von **13** (6,0 g; 1,5 mmol) und NHS (200 mg; 1,7 mmol) in Dichlormethan (80 mL) wird bei 20 bis 25°C DCC (320 mg; 1,6 mmol) gegeben. Anschließend wird das Reaktionsgemisch 24 Stunden bei 20 bis 25°C gerührt. Danach wird die Reaktionsmischung über eine Glasfritte filtriert und das Filtrat im Vakuum eingeengt. Chromatographisch Reinigung an Kieselgel unter Verwendung des Eluenten Dichlormethan/Methanol (90:10) ergibt **16** (5,9 g; 90 %) als farbloses ÖI. Basierend auf diesem Verfahren wurden alle weiteren NHS-Aktivester hergestellt.

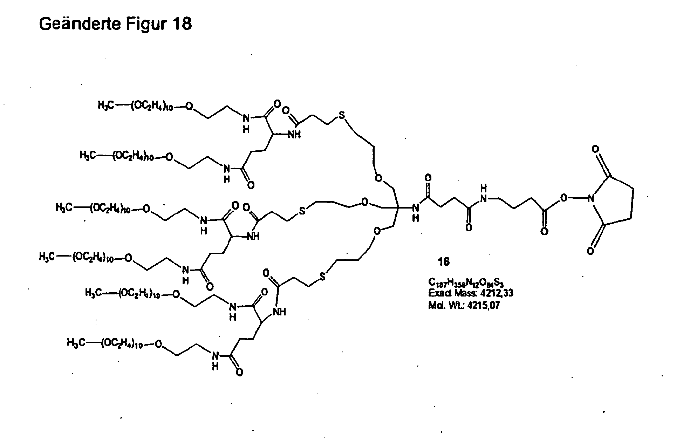

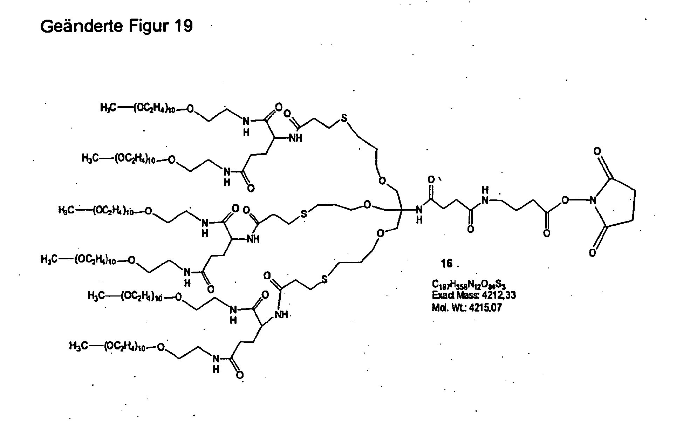

**Die Abbildungen 18 bis 20** zeigen Ausführungsbeispiele für NHS-aktivierte PEG-Reagenzien:
MALDI-MS: m/z 4235,3 [M+Na]⁺; 4251,3 [M+K]⁺; C₁₈₇H₃₅₈N₁₂O₈₄S₃**(****Abb. 18****).**
MALDI-MS: m/z 4249,2 [M+Na]⁺; 4265,2 [M+K]⁺; C₁₈₈H₃₆₀N₁₂O₈₄S₃**(****Abb. 19****).**

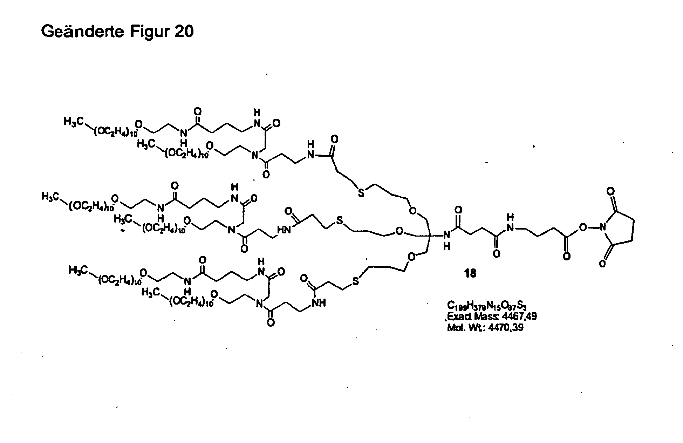

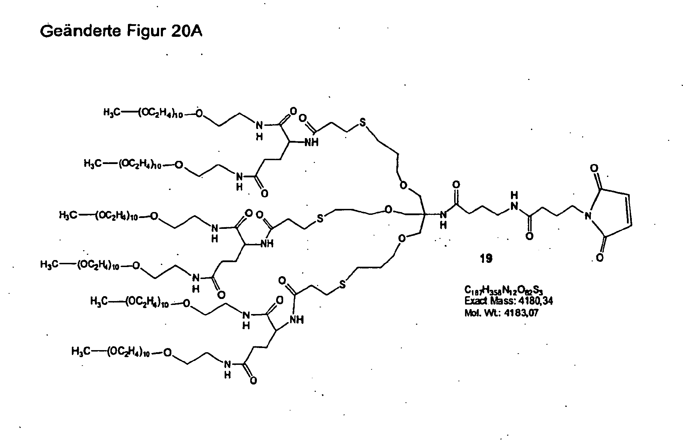

Der Glutarsäure-Linker, der in Verbindung **17** enthalten ist, wird über die Reaktion von **14** mit Glutarsäureanhydrid in Dichlormethan eingeführt. Die entsprechende Carbonsäure, die nach wässriger Aufarbeitung in quantitativer Ausbeute erhalten wird, kann ohne weitere Reinigungsschritte zum NHS-Ester umgesetzt werden.
MALDI-MS: m/z 4490,4 [M+Na]⁺; C₁₉₉H₃₇₉N₁₅O₈₇S₃. **(****Abb. 20****).**

### Allgemeines Verfahren für die Herstellung PEG-Reagenzien mit Maleiimidaktivierung am Beispiel von 19

Zu einer Lösung von **14** (0,41 g; 0,1 mmol) und NHS (5 mg) in Dichlormethan (7 mL) wird bei 20 bis 25°C EDCI (20 mg; 0,1 mmol) gegeben. Anschließend wird das Reaktionsgemisch 10 Stunden bei 20 bis 25°C gerührt. Danach wird die Reaktionsmischung mit Dichlormethan (50 mL) verdünnt. Die resultierende Lösung wird mit verdünnter HCl (10 mL, 0,2 M) gewaschen. Das Einengen der organischen Phase ergibt **19** (0,43 g; quantitativ) als farbloses ÖI.
MALDI-MS: m/z 4203,44 [M+Na]⁺; 4219,42 [M+K]⁺; C₁₈₇H₃₅₈N₁₂O₈₂S₃.

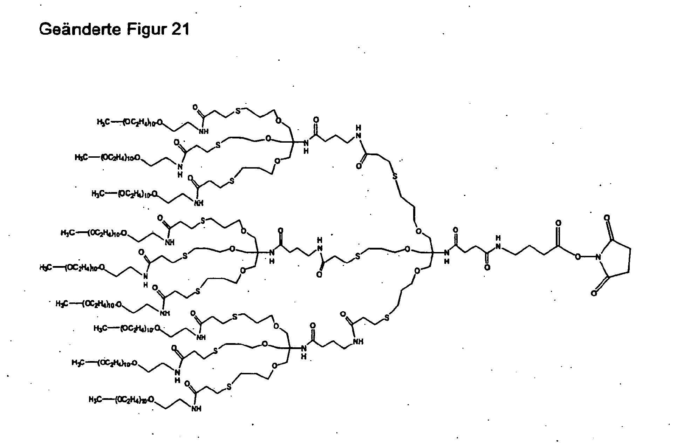

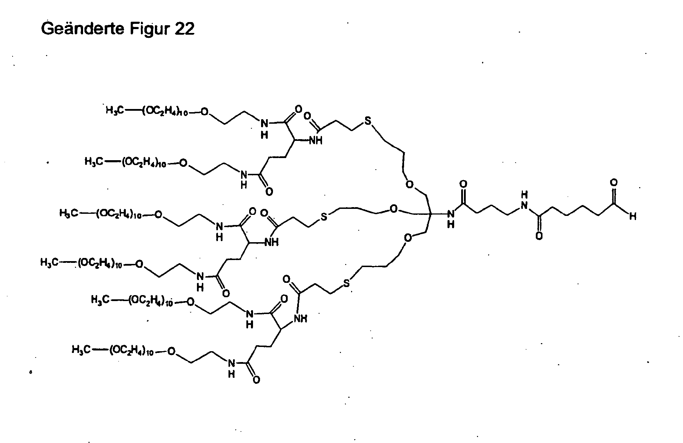

**Die Abbildungen 21 und 22** zeigen weitere Ausführungsbeispiele für bevorzugte PEG-Reagenzien.

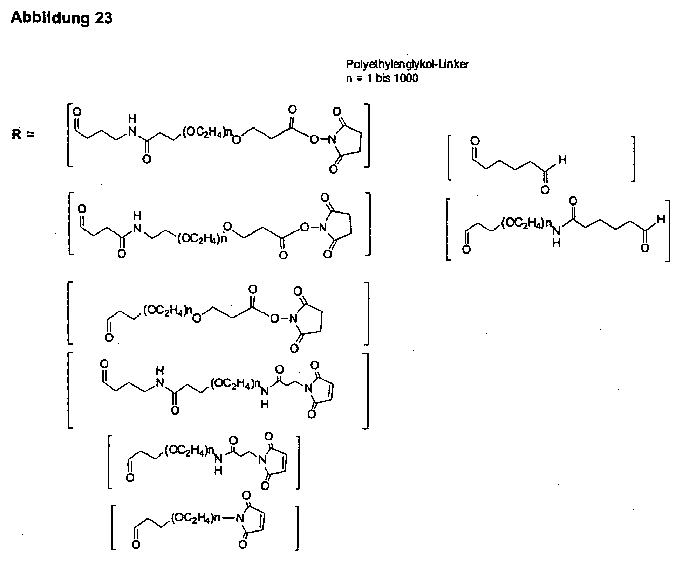

**Abbildung 23** zeigt Linkervariationen und Aktivierungsvariationen.

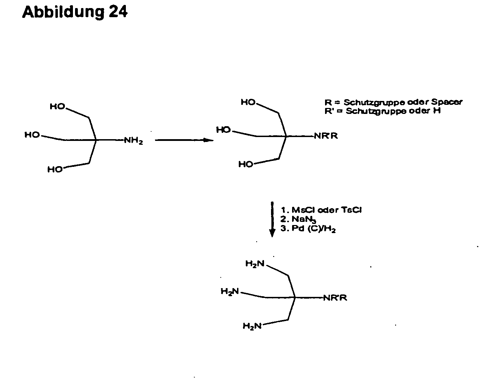

**Abbildung 24** zeigt ein Schema zur Bildung einer bevorzugten Verzweigungskomponente.

## Patentansprüche

1. Verbindung der Formel (I) 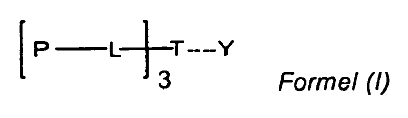 wobei
P und Y jeweils unabhängig voneinander einen Kohlenwasserstoffrest darstellen, welcher Heteroatome enthalten kann,
**dadurch gekennzeichnet,**
**dass** P wenigstens eine Gruppe der Formel (IIa)
R₁-(CH₂-CH₂-O)ₙ-CH₂-CH₂-
aufweist, worin
R₁ H, OH oder ein Kohlenwasserstoffrest mit 1 bis 50 Kohlenstoffatomen ist, welcher Heteroatome enthalten kann,
n bei jedem Auftreten unabhängig eine ganze Zahl von 2 bis 1000 ist, und dass die Gruppe T die Struktur 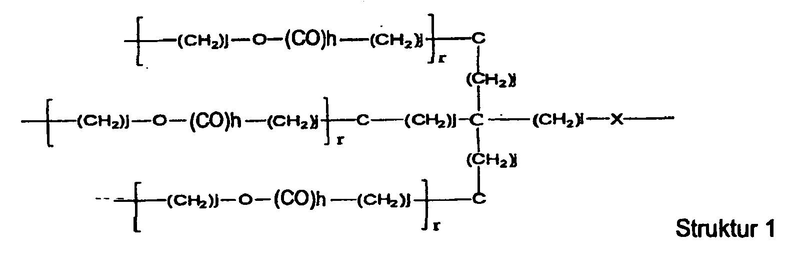 aufweiset, wobei
X NH, C=O, S oder O sein kann
h 0 oder 1 ist
j bei jedem Auftreten unabhängig voneinander eine ganze Zahl zwischen 0 und 10, und
r bei jedem Auftreten unabhängig voneinander eine ganze Zahl zwischen 0 und 3 ist,
und **dass** die Gruppe L die Struktur 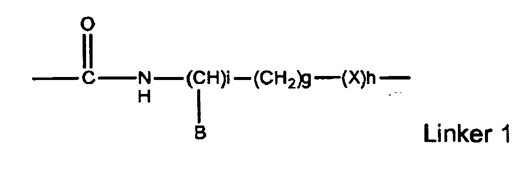 oder 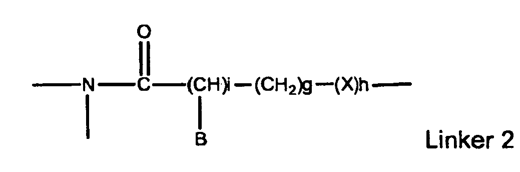 hat,
wobei
B H, OH, C₁-C₄-Alkyl, O-R₂, oder CO-R₃ darstellt,
R₂ bei jedem Auftreten unabhängig einen Kohlenwasserstoffrest mit 1 bis 6 C-Atomen darstellt,
R₃ OH oder NR₄R₅ ist,
R₄ und R₅ jeweils unabhängig H oder einen Kohlenwasserstoffrest, welcher Heteroatome enthalten kann, darstellen, wobei R₄ und R₅ zusammen auch ein Ringsystem bilden können,
i und g jeweils eine ganze Zahl zwischen 1 und 10 ist,
X gleich S und
h 0 oder 1 ist,
wobei wenn L die Struktur Linker 2 hat, P unabhängig voneinander H oder ein Verbindung ist, die eine Gruppe der Formel (IIa) enthält, wobei die Verbindung insgesamt wenigstens drei Gruppen der Formel (IIa) enthält.

2. Verbindung nach Anspruch 1
**dadurch gekennzeichnet,**
**dass** die Bindegruppe Y ausgewählt ist aus Gruppen, die mit einer Aminogruppe, einer Thiolgruppe, einer Carboxylgruppe, einer Guanidingruppe, einer Carbonylgruppe, einer Hydroxylgruppe, einem Heterozyklus, einer C-nukleophilen Gruppe, einer C-elektrophilen Gruppe, einem Phosphat oder einem Sulfat bindefähig sind oder ein Chelat oder einen Komplex mit Metallen bilden können oder eine Bindung an Silicium-haltigen Oberflächen eingehen können.

3. Verbindung nach Anspruch 1 oder 2,
**dadurch gekennzeichnet,**
**dass** sie wenigstens vier Gruppen der Formel (IIa) enthält.

4. Verbindung nach Anspruch 1 oder 2,
**dadurch gekennzeichnet,**
**dass** sie wenigstens fünf Gruppen der Formel (IIa) enthält.

5. Verbindung nach Anspruch 1 oder 2,
**dadurch gekennzeichnet,**
**dass** sie wenigstens sechs Gruppen der Formel (IIa) enthält.

6. Verbindung nach Anspruch 1,
**dadurch gekennzeichnet,**
**dass** mindestens einer der Reste P oder/und L verzweigt ist.

7. Verbindung nach einem der vorhergehenden Ansprüche,
**dadurch gekennzeichnet,**
**dass** der Rest P wenigstens zwei Gruppen der Formel (IIa) enthält.

8. Verbindung nach einem der vorhergehenden Ansprüche,
**dadurch gekennzeichnet,**
**dass** P verzweigt ist und als Verzweigungskomponente eine Aminosäure enthält.

9. Verbindung nach Anspruch 8,
**dadurch gekennzeichnet,**
**dass** die Aminosäure Glutaminsäure ist und die Verbindung der Formel (I) die folgende Struktur aufweist 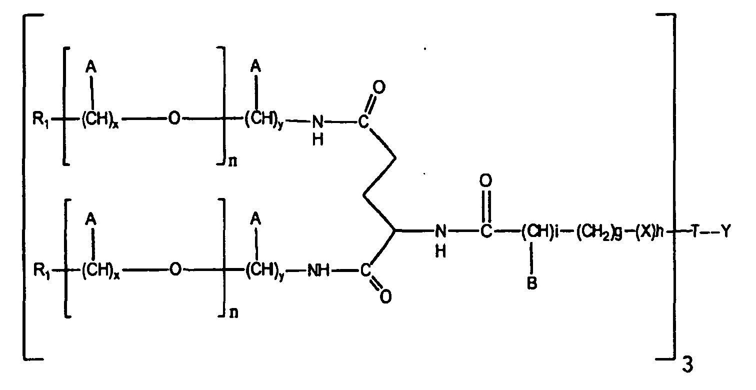 worin
T wie in Anspruch 1 definiert ist,
Y einen Kohlenwasserstoffrest darstellt, welcher Heteroatome enthalten kann,
A bei jedem Auftreten unabhängig H, OH, C₁-C₄-Alkyl, O-R₂ oder CO-R₃ darstellt,
R₁ H, OH oder ein Kohlenwasserstoffrest mit 1 bis 50 Kohlenstoffatomen ist, welcher Heteroatome enthalten kann,
R₂ bei jedem Auftreten unabhängig einen Kohlenwasserstoffrest mit 1 bis 6 C-Atomen darstellt,
R₃ OH oder NR₄R₅ ist,
R₄ und R₅ jeweils unabhängig H oder einen Kohlenwasserstoffrest, welcher Heteroatome enthalten kann, darstellen, wobei R₄ und R₅ zusammen auch ein Ringsystem bilden können,
n bei jedem Auftreten unabhängig eine ganze Zahl von 2 bis 1000 ist und
x bei jedem Auftreten eine ganze Zahl von 1 bis 10 ist,
y eine ganze Zahl von 0 bis 50 darstellt,
B H, OH, C₁-C₄-Alkyl, O-R₂ oder CO-R₃ darstellt,
i und g jeweils eine ganze Zahl zwischen 1 und 10 ist,
X gleich S ist, und
h 0 oder 1 ist.

10. Verbindung nach einem der vorhergehenden Ansprüche,
**dadurch gekennzeichnet,**
**dass** der Rest P mindestens eine Gruppe der Formel (P'-L'-)-T'----Y' enthält,
wobei P', L', T' und Y' jeweils die für P, L, T und Y in den vorhergehenden Ansprüchen angegebenen Bedeutungen aufweisen.

11. Verbindung nach einem der vorhergehenden Ansprüche,
**dadurch gekennzeichnet,**
**dass** der Rest P mindestens eine Gruppe der Formel (IV) 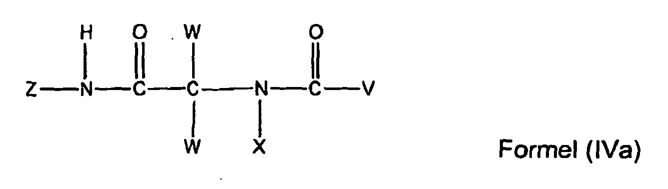 oder 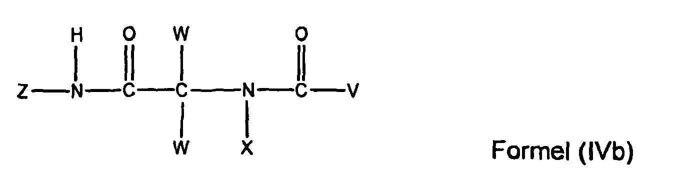 worin
die Reste V, W, X und Z jeweils unabhängig voneinander einen Kohlenwasserstoffrest darstellen, welcher Heteroatome enthalten kann oder/und V, W oder/und X Wasserstoff darstellen.

12. Konjugat, umfassend eine Verbindung der Formel (I), wie in einem der Ansprüche 1 bis 11 definiert, kovalent gebunden an einen biopharmazeutischen, pharmazeutischen oder/und synthetischen Wirkstoff.

13. Konjugat, umfassend eine Verbindung der Formel (I), wie in einem der Ansprüche 1 bis 11 definiert, kovalent gebunden an eine Oberfläche oder/und einen Biokatalysator.

14. Konjugat, umfassend eine Verbindung der Formel (I), wie in einem der Ansprüche 1 bis 11 definiert, kovalent gebunden an ein Enzym.

15. Konjugat, umfassend eine Verbindung der Formel (I), wie in einem der Ansprüche 1 bis 11 definiert, kovalent gebunden an Medizinprodukte oder kovalent gebunden an Hilfsmittel zur Darreichung von Wirkstoffen.

16. Konjugat, umfassend eine Verbindung der Formel (I), wie in einem der Ansprüche 1 bis 11 definiert, kovalent gebunden an Gewebe für Heterotransplantate, Herzklappen, Liposomen oder Nanokapseln.

17. Pharmazeutische Zusammensetzung, umfassend eine Verbindung nach einem der Ansprüche 1 bis 11, 13 oder 15 bis 16 oder ein Konjugat nach einem der Ansprüche 12 oder 14.

18. Diagnostische Zusammensetzung, umfassend eine Verbindung nach einem der Ansprüche 1 bis 11, 13 oder 15 bis 16 oder ein Konjugat nach einem der Ansprüche 12 oder 14.

19. Verwendung eines Konjugats nach Anspruch 12 zur Herstellung eines Arzneimittels zur Behandlung von Krebs oder Herz-Kreislauf-Erkrankungen, Stoffwechselerkrankungen, neuronalen bzw. cerebralen Erkrankungen oder entzündlichen Prozessen und Immun- oder Autoimmumerkrankungen.

20. Verwendung nach Anspruch 19 zur Herstellung eines Arzneimittels zur Behandlung von Alzheimer, Parkinson, Infektionen oder rheumatoider Arthritis.

## Claims

1. A compound of formula (I) 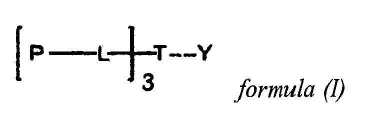 wherein
P and Y each independently represent a hydrocarbon group which may contain heteroatoms,
**characterized in that**
P is at least a group of formula (IIa)
R₁-(CH₂-CH₂-O)ₙCH₂-CH₂-
wherein
R₁ is H, OH or a hydrocarbon group having 1 to 50 carbon atoms which may contain heteroatoms,
n at each occurrence is independently an integer of 2 to 1000,
and group T has the structure 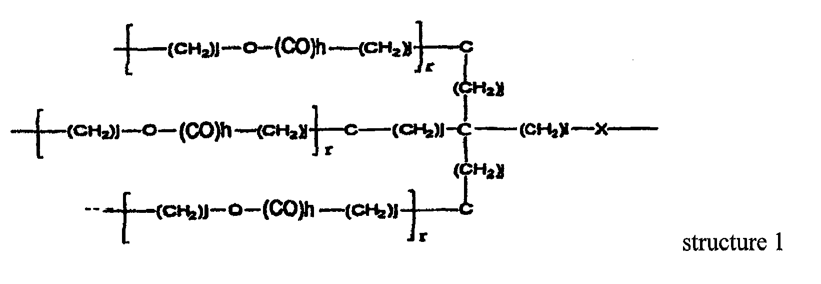 wherein
X may be NH, C=O, S or O,
h is 0 or 1,
j at each occurrence is independently an integer of between 0 and 10, and
r at each occurrence is independently an integer of between 0 and 3,
and group L has the structure 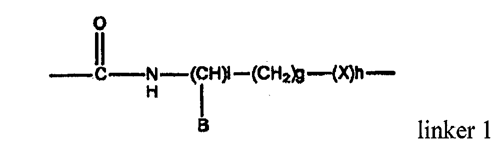 or 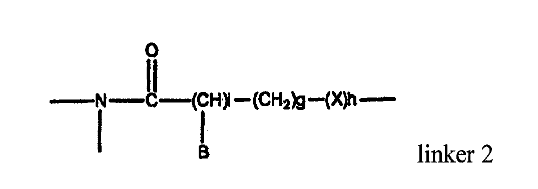 wherein
B represents H, OH, C₁-C₄ alkyl, O-R₂ or CO-R₃,
R₂ at each occurrence independently represents a hydrocarbon group having 1 to 6 C atoms,
R₃ is OH or NR₄R₅,
R₄ and R₅ each independently represent H or a hydrocarbon group which may contain heteroatoms, wherein R₄ and R₅ together may also form a ring system,
i and g each are an integer of between 1 and 10,
X is S, and
h is 0 or 1,
wherein, when L has the structure of linker 2, P is independently H or a compound containing a group of formula (IIa), wherein the compound in total contains at least three groups of formula (IIa).

2. The compound according to claim 1,
**characterized in that**
binding group Y is selected from groups being able to bind with an amino group, a thiol group, a carboxyl group, a guanidine group, a carbonyl group, a hydroxyl group, a heterocycle, a C-nucleophilic group, a C-electrophile group, a phosphate or a sulphate or being able to form a chelate or a complex with metals or being able to form a bond at silicon containing surfaces.

3. The compound according to claim 1 or 2,
**characterized in that**
it contains at least four groups of formula (IIa).

4. The compound according to claim 1 or 2,
**characterized in that**
it contains at least five groups of formula (IIa).

5. The compound according to claim 1 or 2,
**characterized in that**
it contains at least six groups of formula (IIa).

6. The compound according to claim 1,
**characterized in that**
at least one of groups P or/and L is branched.

7. The compound according to one of the preceding claims,
**characterized in that**
group P contains at least two groups of formula (IIa).

8. The compound according to one of the preceding claims,
**characterized in that**
P is branched and contains an amino acid as a branching component.

9. The compound according to claim 8,
**characterized in that**
the amino acid is glutamic acid and the compound of formula (I) has the following structure 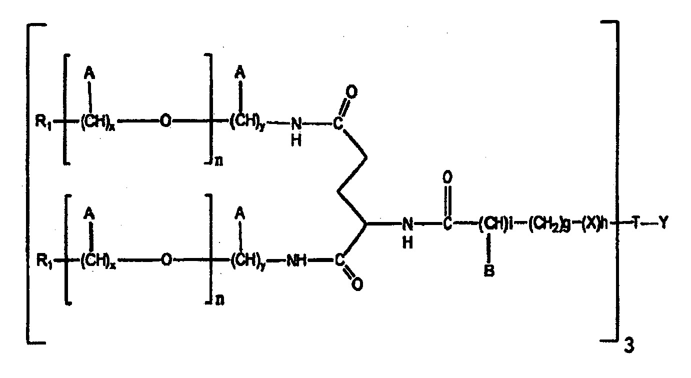 wherein
T is defined as in claim 1,
Y represents a hydrocarbon group which may contain heteroatoms,
A at each occurrence independently represents H, OH, C₁-C₄ alkyl, O-R₂ or CO-R₃,
R₁ is H, OH or a hydrocarbon group having 1 to 50 carbon atoms which may contain heteroatoms,
R₂ at each occurrence independently represents a hydrocarbon group having 1 to 6 C atoms,
R₃ is OH or NR₄R₅,
R₄ and R₅ each independently represent H or a hydrocarbon group which may contain heteroatoms, wherein R₄ and R₅ together may also form a ring system,
n at each occurrence independently is an integer of 2 to 1000, and
x at each occurrence is an integer of 1 to 10,
y represents an integer of 0 to 50,
B represents H, OH, C₁-C₄ alkyl, O-R₂ or CO-R₃,
i and g each are an integer of between 1 and 10,
X is S, and
h is 0 or 1.

10. The compound according to one of the preceding claims,
**characterized in that**
group P at least contains one group of formula (P'-L'-)-T'----Y',
wherein P', L', T' and Y' each have the meanings which are given in the preceding claims for P, L, T and Y.

11. The compound according to one of the preceding claims,
**characterized in that**
group P at least comprises a group of formula (IV) 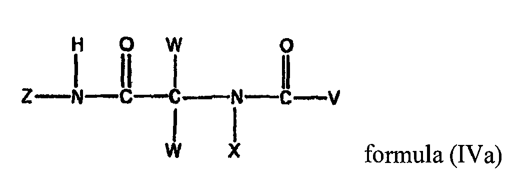 or 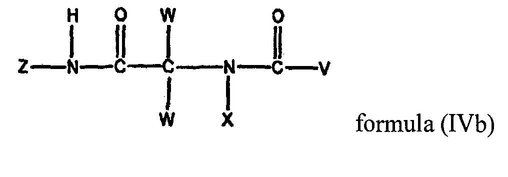 wherein
groups V, W, X and Z each independently represent a hydrocarbon group which may contain heteroatoms or/and V, W or/and X represent hydrogen.

12. A conjugate, comprising a compound of formula (I) as defined in one of claims 1 to 11, covalently bonded to a biopharmaceutical, pharmaceutical or/and synthetic active ingredient.

13. A conjugate, comprising a compound of formula (I) as defmed in one of claims 1 to 11, covalently bonded to a surface or/and a biocatalyst.

14. A conjugate, comprising a compound of formula (I) as defined in one of claims 1 to 11, covalently bonded to an enzyme.

15. A conjugate, comprising a compound of formula (I) as defined in one of claims 1 to 11, covalently bonded to medicine products or covalently bonded to auxiliary agents for the administration of active ingredients.

16. A conjugate, comprising a compound of formula (I) as defined in one of claims 1 to 11, covalently bonded to tissue for xenografts, heart valves, liposomes or nanocapsules.

17. A pharmaceutical composition, comprising one compound according to one of claims 1 to 11, 13 or 15 to 16 or a conjugate according to one of claims 12 or 14.

18. A diagnostic composition, comprising a compound according to one of claims 1 to 11, 13 or 15 to 16 or a conjugate according to one of claims 12 or 14.

19. A use of a conjugate according to claim 12 for the production of a medicament for the treatment of cancer or cardiovascular diseases, metabolic diseases, neuronal and/or cerebral diseases or inflammatory processes and immune or autoimmune diseases.

20. The use according to claim 19 for the preparation of a medicament for the treatment of Alzheimer disease, Parkinson disease, infections or rheumatoid arthritis.

## Revendications

1. Composé de formule (I) :
[P - L -]₃-T--Y formule (I)
où
P et Y représentent chacun indépendamment l'un de l'autre, un reste hydrocarbure, qui peut contenir un hétéroatome,
**caractérisé en ce que**
P présente au moins un radical de formule (IIa) :
R₁- (CH₂-CH₂-O)ₙ-CH₂-CH₂-
où
R₁ est H, OH ou un reste hydrocarbure ayant 1 à 50 atomes de carbone, qui peut contenir un hétéroatome,
n est, indépendamment dans chaque occurrence, un nombre entier allant de 2 à 1000, et
le radical T présente la structure : 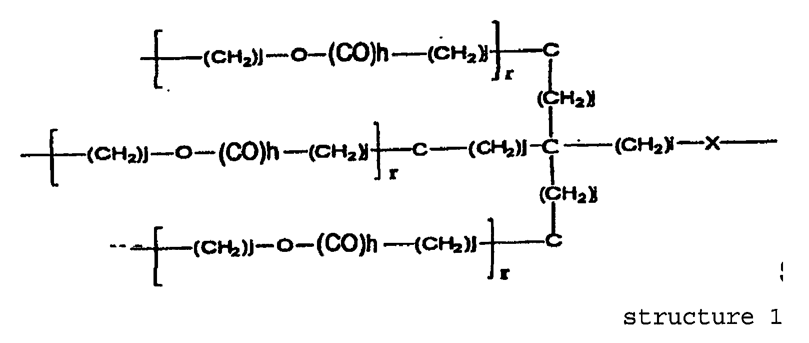 où
X peut être NH, C=O, S ou O,
h est 0 ou 1,
j est, indépendamment dans chaque occurrence, un nombre entier entre 0 et 10, et
r est, indépendamment dans chaque occurrence, un nombre entier entre 0 et 3, et
le radical L a la structure : 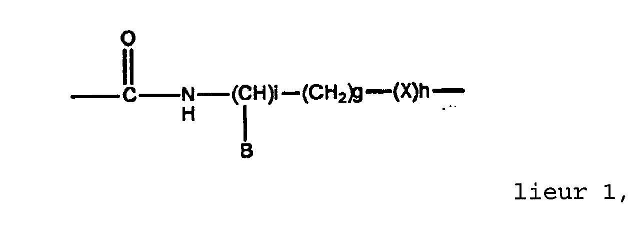 ou 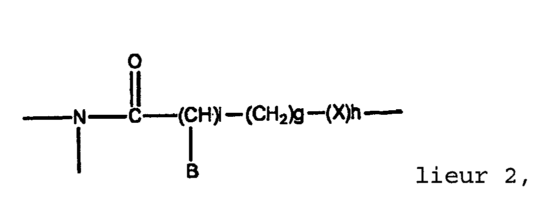 où
B représente H, OH, alkyle en C₁-C₄, O-R₂ ou CO-R₃,
R₂ représente indépendamment dans chaque occurrence, un reste hydrocarbure ayant 1 à 6 atomes C,
R₃ est OH ou NR₄R₅,
R₄ et R₅ représentent chaque fois indépendamment, H ou un reste hydrocarbure, qui peut contenir un hétéroatome, où R₄ et R₅ peuvent former ensemble, un système cyclique,
i et g sont chacun, un nombre entier entre 1 et 10,
X est S, et
h est 0 ou 1,
où lorsque L a la structure du lieur 2, P est indépendamment, H ou un composé, qui contient un radical de formule (IIa), où le composé contient au total, au moins trois radicaux de formule (IIa).

2. Composé selon la revendication 1, **caractérisé en ce que** le radical de liaison Y est choisi parmi les radicaux qui peuvent se lier à un radical amino, un radical thiol, un radical carboxyle, un radical guanidine, un radical carbonyle, un radical hydroxyle, un hétérocycle, un radical C-nucléophile, un radical C-électrophile, un phosphate ou un sulfate, ou qui peuvent former un chélate ou un complexe avec des métaux ou qui peuvent lier avec une surface contenant du silicium.

3. Composé selon la revendication 1 ou 2, **caractérisé en ce qu'**il contient au moins quatre radicaux de formule (IIa).

4. Composé selon la revendication 1 ou 2, **caractérisé en ce qu'**il contient au moins cinq radicaux de formule (IIa).

5. Composé selon la revendication 1 ou 2, **caractérisé en ce qu'**il contient au moins six radicaux de formule (IIa).

6. Composé selon la revendication 1, **caractérisé en ce qu'**au moins un des restes P et/ou L est ramifié.

7. Composé selon l'une des revendications précédentes, **caractérisé en ce que** le reste P contient au moins deux radicaux de formule (IIa).

8. Composé selon l'une des revendications précédentes, **caractérisé en ce que** P est ramifié et qu'il contient comme composant de ramification, un acide aminé.

9. Composé selon la revendication 8, **caractérisé en ce que** l'acide aminé est l'acide glutamique et que le composé de formule (I) présente la structure suivante : 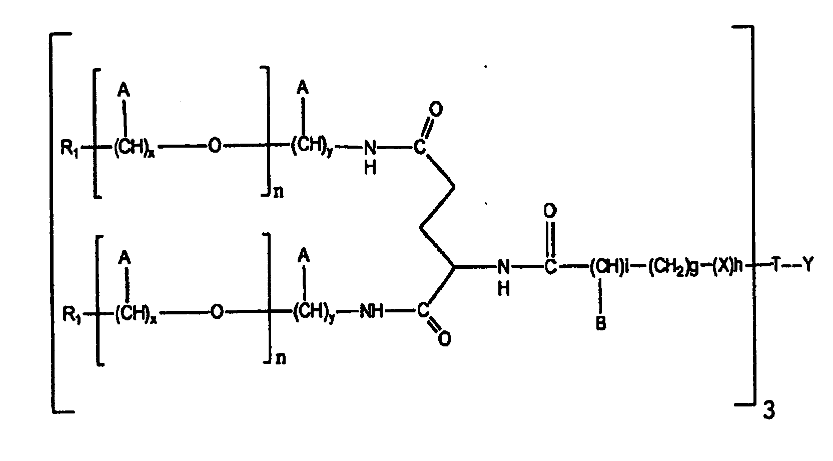 où
T est défini comme à la revendication 1,
Y représente un reste hydrocarboné, qui peut contenir un hétéroatome,
A représente, indépendamment pour chaque occurrence, H, OH, alkyle en C₁-C₄, O-R₂ ou CO-R₃,
R₁ est H, OH ou un reste hydrocarbure ayant 1 à 50 atomes de carbone, qui peut contenir un hétéroatome,
R₂ représente indépendamment pour chaque occurrence, un reste hydrocarbure ayant 1 à 6 atomes C,
R₃ est OH ou NR₄R₅,
R₄ et R₅ représentent chaque fois indépendamment, H ou un reste hydrocarbure, qui peut contenir un hétéroatome, où R₄ et R₅ peuvent former ensemble, également un système cyclique,
n est indépendamment pour chaque occurrence, un nombre entier de 2 à 1000, et
x est pour chaque occurrence, un nombre entier de 1 à 10,
y représente un nombre entier de 0 à 50,
B représente H, OH, alkyle en C₁-C₄, O-R₂ ou CO-R₃,
i et g sont chacun, un nombre entier entre 1 et 10,
X est S, et
h est 0 ou 1.

10. Composé selon l'une des revendications précédentes, **caractérisé en ce que** le reste P contient au moins un radical de formule (P'-L')-T'--Y',
où P', L', T' et Y' présentent chaque fois, les significations données pour P, L, T et Y dans les revendications précédentes.

11. Composé selon l'une des revendications précédentes, **caractérisé en ce que** le reste P contient au moins un radical de formule (IV) : 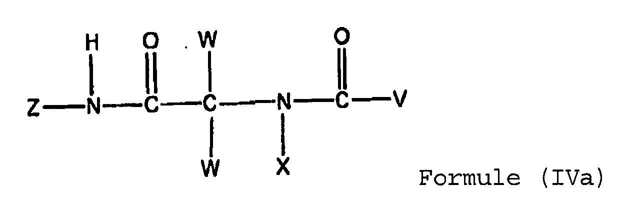 ou 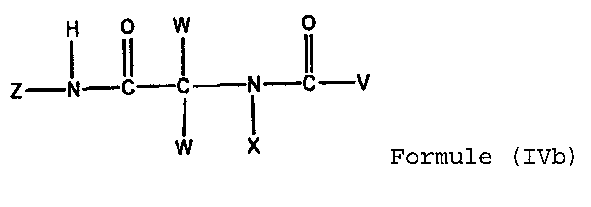 où
les restes V, W, X et Z représentent indépendamment l'un de l'autre, un reste hydrocarbure, qui peut contenir un hétéroatome, et/ou V, W et/ou X représentent l'hydrogène.

12. Conjugué, comprenant un composé de formule (I), comme défini dans l'une des revendications 1 à 11, lié de manière covalente à un agent actif biopharmaceutique, pharmaceutique et/ou synthétique.

13. Conjugué, comprenant un composé de formule (I), comme défini dans l'une des revendications 1 à 11, lié de manière covalente à une surface et/ou un biocatalyseur.

14. Conjugué, comprenant un composé de formule (I), comme défini dans l'une des revendications 1 à 11, lié de manière covalente à une enzyme.

15. Conjugué, comprenant un composé de formule (I), comme défini dans l'une des revendications 1 à 11, lié de manière covalente à un produit médical ou lié de manière covalente à un auxiliaire pour l'administration d'agents actifs.

16. Conjugué, comprenant un composé de formule (I), comme défini dans l'une des revendications 1 à 11, lié de manière covalente à des tissus pour des hétérotransplantations, des valves cardiaques, des liposomes ou des nanocapsules.

17. Composition pharmaceutique, comprenant un composé selon l'une des revendications 1 à 11, 13 ou 15 à 16 ou un conjugué selon l'une des revendications 12 ou 14.

18. Composition diagnostique, comprenant un composé selon l'une des revendications 1 à 11, 13 ou 15 à 16 ou un conjugué selon l'une des revendications 12 ou 14.

19. Utilisation d'un conjugué selon la revendication 12, pour la préparation d'un agent pharmaceutique pour le traitement du cancer ou de maladies cardiovasculaires, de troubles du métabolisme, de maladies neuronales ou cérébrales, ou de processus inflammatoires et de maladies immunes et auto-immunes.

20. Utilisation selon la revendication 19, pour la préparation d'un agent pharmaceutique pour le traitement de la maladie d'Alzheimer, de Parkinson, d'infections ou de la polyarthrite rhumatoïde.
